# EUROPEAN PATENT APPLICATION

(11) **EP 4 702 981 A1**
(43) Date of publication of application: **04.03.2026**
(21) Application number: 24197571.3
(22) Date of filing: 30.08.2024
(51) Int. Cl.: A61K 35/74, A61K 35/76, A61K 38/00, C07K 14/005, C12N 7/00, C12N 9/22, C12N 15/63, C12N 15/70, C12N 15/73, C12N 15/74, C12N 15/86

(54) **TARGETED ANTIBACTERIAL TREATMENT**

(71) Applicant: Københavns Universitet, 1165 København K (DK)
(72) Inventor: MOLIN HØYLAND-KROGHSBO, Nina, 1165 København K (DK); KOONCE, Kira Céline, 1165 København K (DK); JUEL MAURITZEN, Jesper, 1165 København K (DK); LASSEN, Swenja, 1165 København K (DK)
(74) Representative: Zacco Denmark A/S

(57) **Abstract**

The present invention relates to engineered bacteriophages encoding and expressing at least one inhibitor of a quorum-sensing molecule. The bacteriophages of the present invention are useful for reducing or eliminating pathogenic bacteria, such as bacterial infections.

## Description

### Field of the invention

The present invention relates to engineered bacteriophages encoding and expressing at least one inhibitor of a quorum-sensing molecule. The bacteriophage of the present invention are useful for reducing or eliminating pathogenic bacteria, such as bacterial infections. The present invention further relates to methods of using the bacteriophage in targeted antibacterial treatment.

### Background of the invention

By 2050, the death toll by previously preventable or easily curable bacterial infections will surpass that of cancer deaths, unless the further spread of antibiotic resistance is prevented and new therapies are developed. A promising strategy is phage therapy, exploiting bacteriophage (phage) viruses, which are natural enemies of bacteria. However, bacteria fight back, limiting the success of phage therapy. Notably, bacteria rely on cell-cell communication, known as quorum sensing to orchestrate both virulence programs and phage defenses.

The alarming rise of antibiotic resistance thus calls for an urgent need to develop alternative therapies. The increasingly antibiotic resistant pathogenic bacterium *Pseudomonas aeruginosa* is one of the "priority pathogens" that pose the greatest threat to human health. *P. aeruginosa* causes severe disease in chronic cystic fibrosis lung infections and is a major source of hospital acquired infections in *e.g.* burn wound victims and immunocompromised patients. Moreover, *P. aeruginosa* is a versatile pathogen, infecting animals and plants, underscoring its importance for agriculture and food security.

The use of bacteriophage viruses (phages) as an option for killing pathogenic bacteria is reemerging. Phages are viruses that infect, replicate within, and kill bacteria. During one lytic infectious cycle, a phage may produce 100-150 phage progeny, which are released when the bacterium lyses. Alternatively, temperate phages can execute the lysogenic life cycle to integrate their genome into the host chromosome and reside dormant (called a prophage), until conditions in the prophage-containing cell (called a lysogen) favor a switch from lysogenic to lytic replication cycle. Shortly after the discovery of phages over 100 years ago, phage therapy was employed to treat bacterial infections, but poor understanding of phage biology meant therapy had limited success, and it was largely abandoned in the wake of antibiotics, except at key phage therapy institutes such as the George Eliava Institute of Bacteriophages. With the threat of antibiotic resistance, phage therapy is reemerging as a promising alternative. In recent years, phage therapies using natural virulent phages or phages engineered to be exclusively lytic, were approved for compassionate care use. These have saved critically ill patients suffering from multidrug-resistant infections. However, in some cases, the bacteria developed resistance to the phages. Although cocktails of different phages all targeting the same pathogen via different receptors reduce the probability that bacteria evolve resistance to multiple phages simultaneously, multi-phage-resistant bacteria still arise.

The evolutionary arms race between bacteria and phages has caused the development of multiple defense and anti-defense mechanisms. These include primary defenses preventing phages from infecting the bacteria in the first place, and should these defenses fail, the next in line are systems that serve to prevent the phage from taking over the bacterial cell and converting it into a viral factory. Intracellular phage defenses are diverse and plentiful and include the Clustered Regularly Interspersed Short Palindromic Repeat (CRISPR) with its CRISPR-Associated (cas) genes. CRISPR-Cas are adaptive microbial immune systems. Additionally, groups of bacteria can coordinate to form physical defenses or spatial barriers to phage infection.

As expressing defense systems inappropriately is costly, bacteria have developed mechanisms to activate the defenses when necessary. For example, quorum sensing (QS), which is a cell-cell communication mechanism, enables bacteria to monitor cell-population density and behave as though they were a multicellular organism. QS depends on the production, release, and group-wide detection of diffusible quorum sensing signaling molecules (also known as autoinducers, AIs). At high-cell-density, the accumulation of quorum sensing molecules induces bacterial populations to synchronously activate hundreds of genes involved in group behavior, such as virulence genes and those encoding phage defenses.

*P. aeruginosa* communicates using QS to coordinate behavior such as biofilm formation and virulence in addition to launching anti-phage defense systems. As described above, anti-phage defenses, some of which are QS-regulated, pose one of the major biological challenges to phage therapy and biocontrol. *P. aeruginosa* has multiple intertwined QS systems, including two canonical LuxI/R-type QS systems: LasI/R and RhlI/R. LasI/R is conventionally considered at the top of the QS cascade in *P. aeruginosa.* LasI synthesizes 3-Oxo-C12-homoserine lactone (3OC12-HSL), which is detected by LasR. 3OC12-HSL-bound LasR activates transcription of the other QS circuit genes *rhlI*/*R* and *pqsABCDEH*/*R.* RhlI/R, in turn, produces and detects C4-homoserine lactone (C4-HSL). The Pseudomonas quinolone signal (PQS) system produces and detects the AI 2-heptyl-4-quinolone (HHQ) and 2-heptyl-3,4-dihydroxyquinoline (PQS). Collectively, these QS systems control the production of an array of virulence factors including elastase, rhamnolipids, and pyocyanin. Intriguingly, while the Las QS system generally activates the PQS system, phage infection of LasI deficient mutants bypasses this regulatory link and directly activates PQS QS, which is a major regulator of a physical phage defense. Thus, phage infection directly activates PQS QS, which controls virulence factors and phage-defense systems, theoretically limiting successful outcomes of phage therapy.

### Summary of the invention

There is a need in the art for new solutions to overcome the increasingly antibiotic resistant pathogenic bacteria. The object of the present invention is to provide a solution to the problem described herein.

Here, the inventors have combined QS inhibition and phage therapy directly by engineering phages to deliver enzymes that degrade N-acyl homoserine lactones, PQS, HHQ and HQNO in *P. aeruginosa.* The dual-function anti-QS phages replicate within and kill the target bacteria, providing local amplification of the phages while releasing AI-degrading enzymes. The potential of using anti-QS phages to disrupt bacterial communication and simultaneously limit virulence and anti-phage defense activation in *P*. *aeruginosa* is presented herein. Using a *Galleria mellonella* wax moth larvae burn wound infection model, the inventors have quantified the ability of engineered anti-QS phages to save the larvae from succumbing to *P*. *aeruginosa* infection, compared to that of phages that do not interfere with QS. Moreover, the inventors have challenged the robustness of the anti-QS phages to inhibit QS in mixed populations of phage-susceptible and phage-resistant cells, demonstrating the ability of the phages to disarm phage-resistant *P. aeruginosa.* The data highlights the future potential of using anti-QS phages to cure *P. aeruginosa* infections through effective killing and silencing emerging phage-resistant mutants rendering them harmless, thereby improving the effectiveness of phage therapy.

In support of the concept, the inventors have engineered anti-quorum sensing phages against the notorious human pathogen *Pseudomonas aeruginosa.* The engineered phages disclosed herein effectively degrade quorum sensing molecules, simultaneously rendering *P. aeruginosa* avirulent and vulnerable to phage attack, saving *Galleria mellonella* from succumbing to *P. aeruginosa* infection. Moreover, the anti-quorum sensing phages inhibit quorum sensing in mixed populations of phage-susceptible and phage-resistant cells, demonstrating the ability of the phages to disarm phage-resistant *P*. *aeruginosa.* The data highlights the potential of anti-quorum sensing phages to circumvent phage defenses, thus improving the effectiveness of phage therapy.

The object of the present invention is to provide bacteriophages targeted to reduce or eliminate bacteria, in particular pathogenic bacteria, which are resistant to antibiotics. The solution provided with the present invention, is engineered bacteriophages encoding and expressing at least one inhibitor of a quorum-sensing molecule. The bacteriophage of the present invention allows for rapid expression of the inhibitor of a quorum-sensing molecule upon phage infection and at the same time ensures a balanced expression that does not inhibit phage replication.

In a first aspect, the present invention provides a bacteriophage encoding and expressing at least one inhibitor of a quorum sensing molecule, wherein the expression of said at least one inhibitor of a quorum sensing molecule is operably linked to a promoter, wherein said promoter is regulated by a repressor. The bacteriophage is genetically modified to encode and express said at least one inhibitor of a quorum-sensing (QS) molecule.

In another aspect, the present invention provides a composition comprising the bacteriophage of the present invention.

In one aspect, the present invention provides a pharmaceutical composition comprising the bacteriophage according to the invention and at least one pharmaceutical acceptable excipient.

In a further aspect, the present invention provides the bacteriophage of the present invention for use as a medicament. The medicament may be a pharmaceutical composition comprising said bacteriophage and at least one pharmaceutical acceptable excipient.

In one aspect, the present invention provides the bacteriophage or a composition such as a pharmaceutical composition for use in the treatment of a bacterial infection. The present invention further provides methods of treating a bacterial infection, said method comprising the administration of said bacteriophage or composition comprising said bacteriophage in an amount sufficient to reduce or eliminate the bacterial infection.

In a further aspect, the bacteriophage of the present targets a plant pathogenic bacterium. Accordingly, the present invention further provides methods of using said bacteriophage targeting a plant pathogenic bacterium for the treatment of a plant infected with said bacterium. Likewise, the present invention provides the use of said bacteriophage to combat a plant pathogenic bacterium.

### Brief description of the drawings

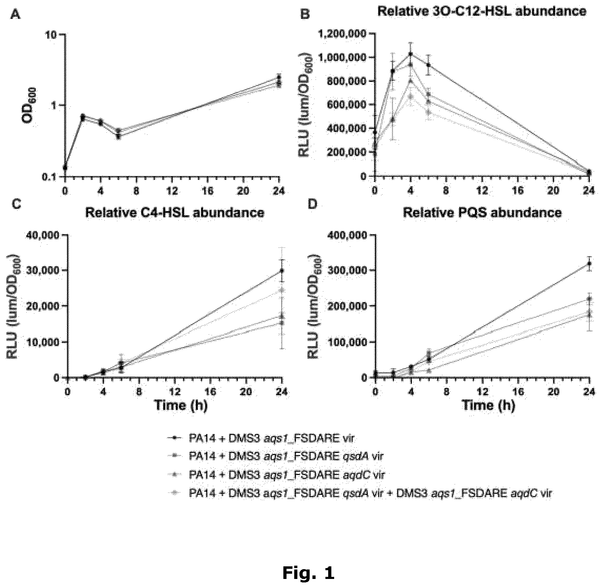

**Figure 1****. Anti-QS phages effectively reduce the accumulation of QS molecules 3O-C12-HSL and PQS.** Cell densities and relative QS molecule abundances of phage-infected *P. aeruginosa* PA14 cultures challenged with DMS3 aqs1_FSDARE vir or anti-QS variants. The phages were mixed in a 1:1 ratio for the dual phage treatment. At OD600~0.14, phage-susceptible PA14 was infected with a 10-fold excess of phages (multiplicity of infection = 10). (A) OD₆₀₀ was measured to monitor bacterial growth post phage infection. The various phage infections inhibited PA14 growth similarly. (B-D) To determine the relative abundance of key QS molecules of the Las, Rhl and PQS system, sterile supernatants were tested using *E. coli* bioreporters. The relative levels of 3O-C12-HSL, C4-HSL, and PQS were quantified as relative light units as a response to these QS molecules. The values are reported as RLU (lum/OD₆₀₀) (relative light units). Whereas the anti-QS phages had no apparent effect on C4-HSL abundance, both 3O-C12-HSL, and PQS levels were significantly reduced in response to anti-QS phage infection. (B) The aqdC-carrying phage as well as the mixture of *aqdC-* and *qsdA*-carrying phages resulted in a 1.9-fold reduction in 3O-C12-HSL 2 h post infection (p = 0.0110 and p = 0.0104 respectively), whereas all anti-QS phages reduced relative 3O-C12-HSL levels 1.4-1.8-fold 6 h post infection (*qsdA:* p = 0.0021, *aqdC:* p = 0.0005, and both phages: p < 0.0001). (D) Anti-QS phages carrying *aqdC* and phage mixtures of *aqdC-* and qsdA-carrying phages lowered the accumulation of PQS (p = 0.0189 and p = 0.0200 respectively, 2 h post infection). Error bars show standard deviation, n = 3. Ordinary one-way ANOVA tests. These data demonstrate that the anti-QS phages effectively suppress accumulation of the QS molecules 3O-C12-HSL and PQS during phage infection.

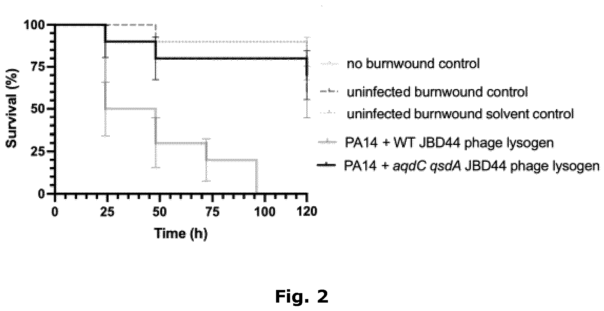

**Figure 2****. Anti-QS prophage rescues *G. mellonella* larvae from infection by** ***P. aeruginosa.** G. mellonella* larvae were either untreated or inflicted with a burn wound and treated as follows: either the wound was untreated, treated with a solvent control, infected with an OD = 1 standardized inoculum of *P. aeruginosa* PA14 JBD44 WT lysogen or a PA14 JBD44 anti-QS lysogen expressing *aqdC* and *qsdA.* Survival was monitored over 120 h. Whereas the JBD44 WT lysogen killed all infected larvae, the JBD44 anti-QS lysogen yielded survival rates comparable to the untreated control (p = 0.0004, log-rank test based on end-point mortality). Error bars show standard deviation, n = 10 larvae per condition. These data demonstrate that virulence is effectively attenuated in the anti-QS phage lysogen, thus protecting *G*. *mellonella* larvae from PA14-mediated infection.

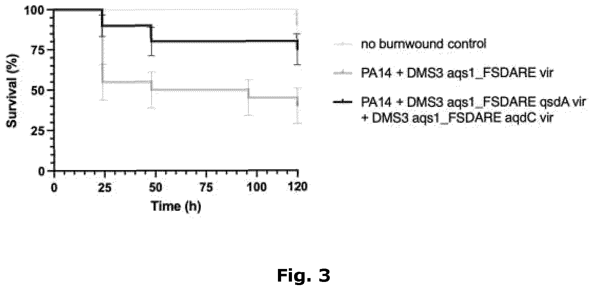

**Figure 3****. Virulent anti-QS phages protect *G. mellonella* larvae from P. *aeruginosa* killing.** *G. mellonella* larvae survival was investigated in response to different phage treatments over a 120-h period. Larvae were either left untreated or inflicted with a burn wound and infected with an undiluted PA14 overnight culture. One hour after bacterial infection, burn wounds were treated with a total of 10⁹ PFU (plaque forming units) of either the parental DMS3 aqsl_FSDARE vir phage or a 1:1 mixture of virulent DMS3 aqsl_FSDARE phages expressing *qsdA* or *aqdC,* respectively. As compared to DMS3 aqsl_FSDARE vir phage treatment resulting in the survival of 40% of the larvae, anti-QS phage treatment led to 75% of larvae surviving infection by PA14 (p = 0.0206, log-rank test based on end-point mortality). Error bars show standard deviation, n = 20 larvae per condition. These data demonstrate that the engineered anti-QS virulent phages significantly reduce *P. aeruginosa* PA14 virulence and protect *G*. *mellonella* larvae from infection.

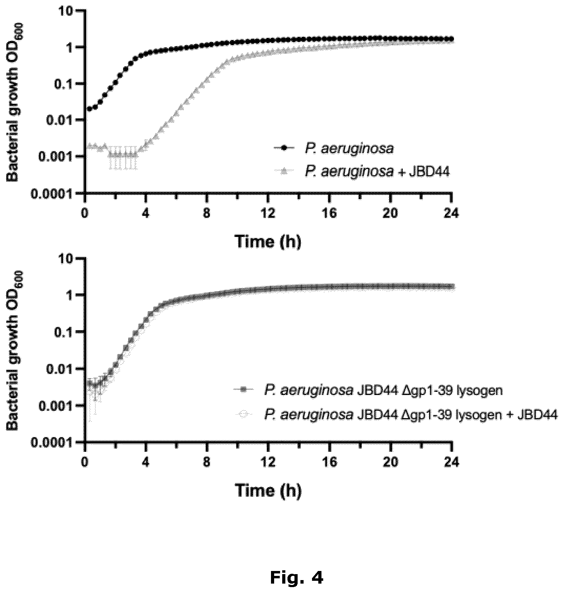

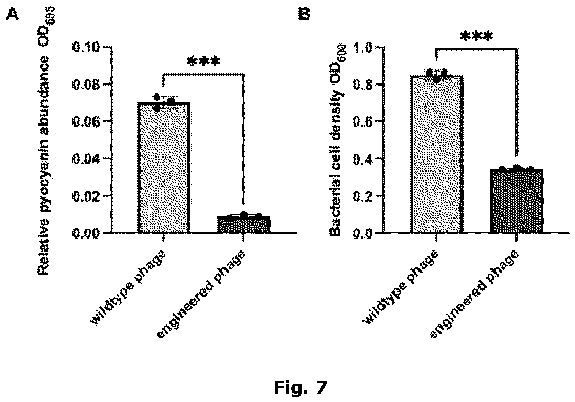

**Figure 4****. *P. aeruginosa* JBD44 Δ*gp1-39* is resistant to JBD44 phage killing.** Bacterial growth of *P. aeruginosa* PA14 and PA14 lysogen JBD44 Δ*gp1-*39 was tested in the presence of a SM buffer solvent control or with an multiplicity of infection (MOI) of 10 of WT JBD44 phage. OD₆₀₀ was measured at 37 °C in a plate reader over a 24 h period. Whereas PA14 growth was noticeably affected in the presence of the WT JBD44 phage, WT JBD44 phage infection did not affect growth of lysogen JBD44 Δ*gp1-39.* Error bars show standard deviation, n = 2. These data show that the JBD44 Δ*gp1-39* prophage provides resistance to JBD44 phage infection. PA14 JBD44 Δ*gp1-39* lysogen was utilized as a phage-resistant mutant in the experiments involving JBD44 phage infection of phage-susceptible and phage-resistant co-cultures (Fig. 7).

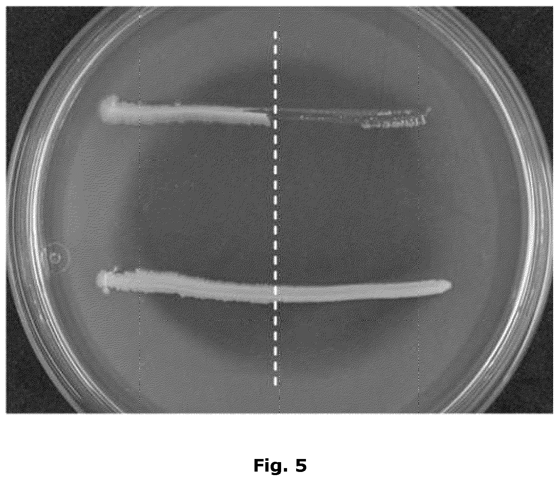

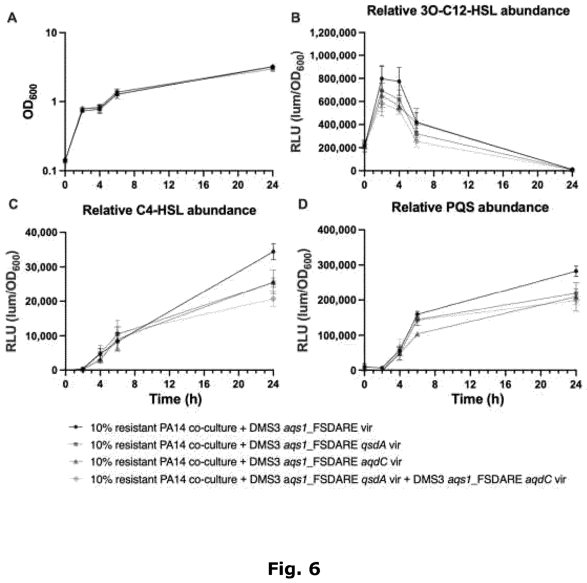

**Figure 5****. *P. aeruginosa* DMS3 aqsl_FSDARE Δ*gp26-48* lysogen is resistant to DMS3 vir killing.** PA14 WT and the PA14 DMS3 aqs1_FSDARE Δ*gp26-48* lysogen were tested in a phage cross-streak assay to determine sensitivity toward the DMS3 vir phage. A drop of DMS3 vir phage solution was added to the center of the agar plate to cover the dotted vertical line. To test phage sensitivity, PA14 WT (top streak) and PA14 DMS3 aqsl_FSDARE Δ*gp26-*48 lysogen (bottom streak) were streaked left to right, resulting in phage exposure on the right side of the plate. Whereas PA14 WT was killed by the DMS3 vir phage, PA14 DMS3 *aqs1*_FSDARE Δ*gp26-48* lysogen growth was unaffected. These data illustrate that the DMS3 aqs1_FSDARE Δ*gp26-48* prophage provides resistance to DMS3 phage killing. PA14 DMS3 aqsl_FSDARE Δ*gp26-48* lysogen was utilized as a phage-resistant mutant in the experiments involving DMS3 phage infection of phage-susceptible and phage-resistant co-cultures (Fig. 6).
**Figure 6****. Anti-QS phages effectively limit QS molecule accumulation in a mixed population of phage-susceptible and phage-resistant *P*. *aeruginosa* cells.** The effect of DMS3 phage infections on bacterial growth and relative QS molecule abundance of a PA14 co-culture consisting of 90% phage-susceptible (PA14 WT) and 10% phage-resistant cells (PA14 DMS3 *aqs1*_FSDARE Δ*gp26-48* lysogen) was tested. Phage treatments include the control phage, a parental DMS3 *aqs1*_FSDARE vir phage, as well as phages expressing either of the anti-QS enzymes, or a 1:1 mixture of *aqdC-* and *qsdA-*expressing phages. At OD600~0.14 PA14 co-cultures were infected with a 10-fold excess of phages (multiplicity of infection = 10). (A) OD₆₀₀ was measured to monitor bacterial growth post phage infection. All the phage variants inhibited the co-culture growth similarly. (B-D) The relative abundance of 30-C12-HSL, C4-HSL, and PQS were quantified by *E. coli* bioreporters producing relative light units as a response to high concentrations of the QS molecules. The values are reported as RLU (lum/OD₆₀₀) (relative light units). Anti-QS phages had no direct effect on C4-HSL abundance whereas 3O-C12-HSL and PQS levels were significantly reduced in response to anti-QS phage infection. (B) Anti-QS phages carrying *aqdC* as well as phage mixtures of *aqdC-* and qsdA-carrying phages lowered the accumulation of 3O-C12-HSL 1.4-fold (p = 0.0233 and p = 0.0103 respectively, 4 h post infection). (D) All anti-QS phages lowered accumulation of PQS (all anti-QS phages p < 0.0001, 2 h post infection). Ordinary one-way ANOVA tests. Error bars show standard deviation, n = 3. These data highlight that even in populations enriched with phage-resistant cells, the anti-QS phages efficiently inhibit the accumulation of 3O-C12-HSL and PQS QS molecules.
**Figure 7****. Anti-QS phages significantly reduce pyocyanin virulence factor production and show enhanced lytic activity in a mixed population of phage-susceptible and phage-resistant *P. aeruginosa* cells.** A mixed population of 90% phage-susceptible (WT PA14) and 10 % phage-resistant (PA14 JBD44 Δ*gp1-39* lysogen) cells at OD600 = 0.1 was challenged with a 5-fold excess (multiplicity of infection of 5) of WT JBD44 or anti-QS JBD44 phage expressing *qsdA* and *aqdC.* 5.5 hours post infection supernatants were sampled and tested for pyocyanin abundance and cell density. (A) As compared to the WT JBD44, the anti-QS JBD44 phage significantly reduced pyocyanin production by 7.8-fold. [Welch's t test; p = 0.0003. Error bars show standard deviation, n = 3.] (B) Compared to the WT phage, OD₆₀₀ was significantly reduced in response to anti-QS phage treatment. [Welch's t test; p = 0.0004. Error bars show standard deviation, n = 3.]. Overall, these data demonstrate that the JBD44 anti-QS phage effectively impairs virulence factor production while inhibiting bacterial growth of mixed PA14 populations.

**Table 2. Anti-QS phages block phage-infection mediated stress response.** Swarm assays were performed with *P. aeruginosa* PA14 inoculated at the center of the swarm and PA14 infected with either DMS3 aqs1_FSDARE vir, DMS3 *aqs1*_FSDARE vir *qsdA* or a 1:1 mixture of *aqdC-* and *qsdA-*expressing phages. Phage infection causes a PQS stress response, resulting in repulsion of uninfected tendrils from the center swarm. This is evident in response to DMS3 *aqs1*_FSDARE vir infection, where 0/4 infected satellite colonies were invaded by tendrils. However, in response to infection by all three phages expressing either *qsdA, aqdC,* or both, tendrils from the center swarm collide with all infected colonies, demonstrating that the anti-QS phages block the PQS induced tendril repulsion, which allows for more efficient phage transmission. In one of the four replicates, the tendrils approaching the DMS3 *aqs1*_FSDARE vir *qsdA*-infected colony swarmed insufficiently, so it could not be determined whether the tendrils would have collided with the colony. These data demonstrate that the anti-QS phages effectively inhibit the phage-infection induced physical phage defense.

### Detailed description of the invention

In describing the embodiments of the invention specific terminology will be resorted to for the sake of clarity. However, the invention is not intended to be limited to the specific terms so selected, and it is understood that each specific term includes all technical equivalents, which operate in a similar manner to accomplish a similar purpose.

When describing the embodiments of the present invention, the combinations and permutations of all possible embodiments have not been explicitly described. Nevertheless, the mere fact that certain measures are recited in mutually different dependent claims or described in different embodiments does not indicate that a combination of these measures cannot be used to advantage. The present invention envisages all possible combinations and permutations of the described embodiments.

As presented herein, the inventors discovered that QS inhibition and phage targeting a pathogenic bacterium is a solution to the problem relating to combating pathogenic bacteria, which are resistant to conventional treatment such as antibiotics. The solution provided herein is a genetically modified bacteriophage expressing a heterologous inhibitor of QS sensing molecules. The anti-quorum sensing phage is modified to ensure rapid expression upon phage infection and at the same time ensures a balanced expression that does not inhibit phage replication. Thus, the design of the phage allows for high expression of the QS inhibitor shortly after infection of the bacterium without impairing the replication of the phage in the infected bacterium. This dual-arm strategy thus includes (i) targeting the pathogenic bacteria by means of causing lysis and in the same time (ii) targeting pathogenic bacteria that is or has become resistant to infection by the bacteriophage by inhibiting QS and thus rendering them avirulent.

As mentioned, the anti-quorum sensing phage is modified to ensure rapid expression upon phage infection, but the expression does not violate phage replication. In order to achieve this, any promoter may be used that ensures rapid temporarily expression upon phage infection and decreasing expression such that phage replication is not impaired. Examples of such promoter is a promoter with a negative feedback mechanism such as a promoter/repressor system, where the activity of the promoter induces the expression of a repressor of the promoter (e.g. encoded in an operon comprising the promoter), which down regulate the activity of the promoter. A bacteriophage modified to include an expression cassette comprising a promoter regulating the expression of a sequence encoding the QS molecule inhibitor and a repressor of the promoter may thus allow for rapid onset of expression of the QS molecule inhibitor, which is subsequently downregulated by the repressor to maintain capability of phage replication.

### Anti-QS bacteriophage

The present invention provides a bacteriophage encoding and expressing at least one inhibitor of a quorum sensing molecule, wherein the expression of said at least one inhibitor of a quorum sensing molecule is operably linked to a promoter, wherein said promoter is regulated by a repressor.

In one embodiment, said promoter regulates transcription of said at least one inhibitor of a quorum sensing molecule and said repressor. Typically, the QS inhibitor and the repressor is encoded by the same transcript whereby the expression of the repressor generates a negative feedback on the promoter activity.

The inventors have demonstrated that the anti-QS bacteriophage of the present invention is more efficient than its counterpart, which does not have anti-QS capabilities in treating a *P. aeruginosa* infection (*see* Examples and **Fig. 2-3**).

In one embodiment, the at least one inhibitor of a quorum sensing molecule and said repressor are encoded by a bi-cistronic or poly-cistronic transcript.

The repressor may be any suitable repressor in the sense that repressor is capable of downregulating the activity of the promoter operably linked to and regulating the expression of the QS inhibitor.

An example of a suitable promoter is an anti-CRISPR promoter (Pacr), which initiates transcription immediately after phage infection. The anti-CRISPR promoter (Pacr) is one of the strongest expressed and most rapidly expressed promoters known. Transcripts are detectable a few minutes after phage infection and accumulates 100-fold within the first 20 min of infection followed by stabilization of transcript levels.

Thus, in one embodiment, the promoter directs expression of the transcript encoding the QS inhibitor within 5 minutes after infection and stabilizes about 100-fold higher about 20 minutes after the infection.

In one particular embodiment, the promoter is an anti-CRISPR promoter (Pacr). In another embodiment, the promoter comprises the sequence set forth in SEQ ID NO: 1 or a functional variant of said sequence. In the context of the present invention, the variant of the promoter is a promoter that displays the same activity or essentially the same activity as the anti-CRISPR promoter (Pacr) and is subject to the same regulation including subject to regulation by a repressor. In one embodiment, the variant promoter comprises a sequence having a sequence identity of at least 80% to the sequence of SEQ ID NO: 1, such as at least 85%, for example at least 90%, such as at least 95%, for example at least 97%, such as at least 98%, for example 99% to the sequence of SEQ ID NO: 1.

In order to regulate the activity of the anti-CRISPR promoter, the bacteriophage may advantageously encode and express an anti-CRISPR associated repressor (Aca protein). The Aca protein binds and represses the Pacr promoter and maintains the ability of the bacteriophage to replicate in the host cell. Accordingly, in one embodiment, the promoter is an anti-CRISPR promoter and said repressor is an anti-CRISPR associated repressor.

In one embodiment, the anti-CRISPR repressor is encoded by a polynucleotide sequence comprising the sequence set forth in SEQ ID NO:2 or a variant of said sequence. In the context of the present invention, the variant of the repressor is a repressor that displays the same activity or essentially the same activity as said anti-CRISPR associated repressor. In one embodiment, the variant sequence encoding the anti-CRISPR associated repressor comprises a sequence having a sequence identity of at least 80% to the polynucleotide sequence of SEQ ID NO: 2, such as at least 85%, for example at least 90%, such as at least 95%, for example at least 97%, such as at least 98%, for example 99% to the polynucleotide sequence of SEQ ID NO: 2.

In one embodiment, the bacteriophage comprises a promoter comprising the sequence set forth in SEQ ID NO: 1 and encodes an anti-CRISPR repressor, wherein the sequence encoding the anti-CRISPR repressor comprises the sequence set forth in SEQ ID NO:2.

In another embodiment, the anti-CRISPR repressor comprises the amino acid sequence set forth in SEQ ID NO: 3 or a functional variant of said sequence. The variant of the repressor is a repressor that displays the same activity or essentially the same activity as said anti-CRISPR associated repressor. In one embodiment, the variant anti-CRISPR associated repressor comprises an amino acid sequence having a sequence identity of at least 80% to the sequence of SEQ ID NO: 3, such as at least 85%, for example at least 90%, such as at least 95%, for example at least 97%, such as at least 98%, for example 99% to the sequence of SEQ ID NO: 3.

The bacteriophage of the present invention may comprise an operon comprising the promoter operably linked to the sequences encoding the QS inhibitor and the sequences encoding the repressor and optionally also a sequence encoding an anti-CRISPR (acr) protein.

In one embodiment, the bacteriophage of the present invention comprises an anti-CRISPR operon comprising an anti-CRISPR promoter and encoding said at least one inhibitor of a quorum sensing molecule and a sequence encoding an anti-CRISPR associated repressor.

In one embodiment, the bacteriophage further encodes and expresses an anti-CRISPR (acr) protein. In one embodiment, the bacteriophage comprises the polynucleotide sequence set forth in SEQ ID NO: 8, encoding an acr protein. In another embodiment, the bacteriophage further encodes and expresses an acr protein comprising the amino acids sequence set forth in SEQ ID NO: 9.

CRISPR-Cas adaptive immune systems protect bacteria and archaea against their invading bacteriophages. Anti-CRISPR proteins interact with specific components of CRISPR-Cas systems, such as the effector nucleases, to avoid the destruction of the phage DNA. Thus, in the context of the present invention, the encoded anti-CRISPR (acr) protein is a defence mechanism to avoid inactivation of the bacteriophage in the host bacterium.

The expression of the anti-CRISPR (acr) protein is preferably regulated by the same promoter that direct the expression of the QS inhibitor. Thus, in one embodiment, the at least one inhibitor of a quorum sensing molecule, said repressor and said an anti-CRISPR (acr) protein are encoded by a poly-cistronic transcript.

The anti-CRISPR operon encodes an anti-CRISPR (acr) protein and an anti-CRISPR repressor and the expression is directed by the anti-CRISPR promoter (Pacr). The sequence encoding the QS inhibitor may be inserted in the anti-CRISPR operon under control of the anti-CRISPR promoter. In one embodiment, the nucleotide sequence encoding the at least one inhibitor of a quorum sensing molecule is inserted in the operon replacing or in part replacing the sequence encoding an endogenous anti-crispr (acr) protein. In one embodiment, the nucleotide sequence encoding the at least one inhibitor of a quorum sensing molecule is inserted in the operon such that the expression of the endogenous anti-crispr (acr) protein is maintained.

In one embodiment, the bacteriophage comprises an endogenous anti-CRISPR operon, wherein said operon is genetically modified to encode and express said at least one inhibitor of a quorum sensing molecule. Thus, in this embodiment, the sequence encoding the QS inhibitor is inserted an endogenous anti-CRISPR operon, where the expression of the endogenous anti-crispr (acr) protein is maintained or the sequence may be inserted to replace (or partly replace) the sequence encoding the endogenous anti-crispr (acr) protein.

The anti-CRISPR operon may also be exogenous. Thus, the exogenous operon may be artificial, an operon from another species of bacteriophage or a modified version of the operon of the bacteriophage used for the preparation of the bacteriophage of the present invention. In one embodiment, the bacteriophage comprises an exogenous anti-CRISPR operon, wherein said operon is genetically modified to encode and express said at least one inhibitor of a quorum sensing molecule.

In one embodiment, the bacteriophage is lytic. In another embodiment, the bacteriophage is temperate. The temperate bacteriophages is typically integrated in the host genome (as a prophage) remains in a lysogenic state until a lytic life cycle is induced. Thus, the temperate bacteriophage maintains the capacity of lysing the bacterium.

The bacteriophage of the present invention is typically provided using recombinant techniques well known known to one skilled in the art. Thus in a preferred embodiment, the bacteriophage is recombinant. The bacteriophage of the present invention may be based on any suitable bacteriophage for infecting a pathogenic bacterium as described herein. Thus, in one embodiment, the bacteriophage of the present invention is recombinant.

Thus the backbone used for the preparation of the bacteriophage of the present invention may be a bacteriophage selected from the list consisting of Phage DMS3 (accession_no.NC_008717.1), Phage JBD44 (accession_no.NC_030929.1), and phages classified in the orders of *Caudoviricetes, Kalamavirales, Mindivirales, Timlovirales, Tubulavirales, Vinavirales,* and *Durnavirales,* and unclassified or reclassified phages.

In one embodiment, the backbone used for the preparation of the bacteriophage of the present invention comprises the sequence of SEQ ID NO: 10 (Phage DMS3). In another embodiment, the backbone used for the preparation of the bacteriophage of the present invention comprises the sequence of SEQ ID NO: 11 (Phage JBD44).

One further aspect of the present invention provides a method for the preparation of a bacteriophage according to the present invention, said method comprising the step of introducing at least one polynucleotide sequence encoding an inhibitor of a quorum sensing molecule in the genome of a bacteriophage, wherein said sequence is inserted in the genome to be operably linked to a promoter, wherein said promoter is regulated by a repressor.

In one embodiment, promoter is an anti-CRISPR promoter and the repressor is an anti-CRISPR associated repressor (Aca protein). In another embodiment, the bacteriophage (cloning bacteriophage) used for the preparation of the bacteriophage of the present invention comprises an anti-CRISPR operon comprising an anti-CRISPR promoter and encoding said at least one inhibitor of a quorum sensing molecule and a sequence encoding an anti-CRISPR associated repressor. In one embodiment, the bacteriophage (cloning bacteriophage) used for the preparation of the bacteriophage of the present invention comprises the polynucleotide sequence of SEQ ID NO: 12 (cloning phage DMS3 (DMS3 aqs1_FSDARE)).

In one embodiment, the bacteriophage of the present invention comprises the sequence set forth in SEQ ID NO: 13 (Phage_DMS3_aqsl_FSDARE_vir_qsdA). In another embodiment, the bacteriophage of the present invention comprises the sequence set forth in SEQ ID NO: 14 (Phage_DMS3_aqsl_FSDARE_vir_aqdC).

In one embodiment, the bacteriophage of the present invention comprises the sequence set forth in SEQ ID NO: 15 (Phage JBD44 Δgp48_53_gp46-47: : Pacr-qsdA-aqdC-aca-T1).

In one embodiment, the bacteriophage of the present invention is a prophage. In another embodiment, the bacteriophage of the present invention comprises the sequence set forth in SEQ ID NO: 16 (Prophage_JBD44_Δgp48-53_gp46-47: : Pacr-qsdA-aqdC-T1)

### Encoded quorum sensing molecule inhibitor

The bacteriophage of the present invention is modified to encode and express at least one quorum sensing molecule inhibitor (also referred to as QS inhibitor herein).

In one embodiment, the bacteriophage encodes and expresses one quorum sensing molecule inhibitor. In another embodiment, the bacteriophage encodes and expresses two quorum sensing molecule inhibitors. In a further embodiment, bacteriophage encodes and expresses a plurality of quorum sensing molecule inhibitors.

In one embodiment, said at least one quorum sensing molecule inhibitor is selected from the group consisting of quorum sensing molecule inhibitor degrading enzymes, and a repressor of said quorum sensing. In embodiments where the bacteriophage encodes more than one quorum sensing molecule inhibitor, the quorum sensing molecule inhibitors are independently selected from the group consisting of quorum sensing molecule inhibitor, degrading enzymes, and a repressor of said quorum sensing.

In one embodiment, said at least one quorum sensing molecule inhibitor is a biological inhibitor that interferes directly with the quorum sensing molecule. In the context of the present invention, a biological inhibitor is a biomolecule that can be encoded and expressed by the bacteriophage. A QS inhibitor may also be a biological molecule that competitively inhibit the QS signaling system.

In one embodiment, the quorum sensing molecule inhibitor is a repressor of the quorum sensing molecule. In another embodiment, the inhibitor of the quorum sensing molecule is a synthase synthesizing a repressor of said quorum sensing molecule, an antisense RNA or CRISPRi repressing production of said quorum sensing molecule.

In one embodiment, said at least one quorum sensing molecule inhibitor is an enzyme, such as an QS degrading enzyme. The enzyme may directly or indirectly inhibit the function of quorum sensing molecule such as by degrading or partly degrading the quorum sensing molecule. In other embodiments, the enzyme degrades or partly degrades a molecule that interact with the quorum sensing molecule and is essential for its function. In another embodiment, the quorum sensing molecule inhibitor is an enzyme that break up outer membrane vesicles that carry QS molecules (such as PQS and long chain homoserine lactones).

In a further embodiment, the at least one quorum sensing molecule inhibitor is an enzyme selected from the group consisting of quorum sensing molecule degrading enzymes such as alkylquinolone degradation C (AqdC), autoinducing peptide (AIP)-degrading protease, AHL-degrading enzymes such as AHL lactonases and acylases (autoinducer inactivation A (AiiA), autoinducer inactivation B (AiiB), autoinducer inactivation C (AiiC), autoinducer inactivation D (AiiD), autoinducer inactivation S (AiiS), attachment gene M (AttM), N-acylhomoserine lactone D (AhlD), N-acylhomoserine lactone K (AhlK), N-acylhomoserine lactone M (AhlM), N-acylhomoserine lactone S (AhlS), metallo-β-lactamase superfamily lactonase (AaL), quorum-sensing signal degradation A (QsdA), quorum-sensing signal degradation H (QsdH), autoinducers degrading hydrolase (AidH), phosphotriesterase-like lactonase (MCP), quorum-quenching lactonase from *Geobacillus kaustophilus* (GKL), NAHL-lactonase (QlcA), quorum signal utilization and inactivation protein (QuiP), pyoverdine synthesis Q (PvdQ), AHL acylase A (HacA), Phosphotriesterase-Like Lactonase SacPox, lactonase SsoPox, and AHL acylase B (HacB).

In one embodiment, the quorum sensing molecule inhibitor is quorum-sensing signal degradation A (QsdA) or a functional variant of QsdA. In another embodiment, the at least one quorum sensing molecule inhibitor comprises the amino acid sequence set forth in SEQ ID NO: 6 (QsdA) or a functional variant thereof. The variant of the QsdA is a protein that displays the same activity or essentially the same activity or higher activity than QsdA. In one embodiment, the variant QsdA comprises an amino acid sequence having a sequence identity of at least 80% to the sequence of SEQ ID NO: 6, such as at least 85%, for example at least 90%, such as at least 95%, for example at least 97%, such as at least 98%, for example 99% to the sequence of SEQ ID NO: 6.

In another embodiment, the bacteriophage comprising the polynucleotide sequence encoding said at least one quorum sensing molecule inhibitor comprises the sequence set forth in SEQ ID NO: 4 (encoding QsdA) or a functional variant thereof. In one embodiment, the variant sequence encoding QsdA comprises a polynucleotide sequence having a sequence identity of at least 80% to the polynucleotide sequence of SEQ ID NO: 4, such as at least 85%, for example at least 90%, such as at least 95%, for example at least 97%, such as at least 98%, for example 99% to the polynucleotide sequence of SEQ ID NO: 4.

In one embodiment, the quorum sensing molecule inhibitor is alkylquinolone degradation C (AqdC) or a functional variant of AqdC. In another embodiment, the at least one quorum sensing molecule inhibitor comprises the amino acid sequence set forth in SEQ ID NO: 7 (AqdC) a functional variant thereof. The variant of the AqdC is a protein that displays the same activity or essentially the same activity or higher activity than AqdC. In one embodiment, the variant AqdC comprises an amino acid sequence having a sequence identity of at least 80% to the sequence of SEQ ID NO: 7, such as at least 85%, for example at least 90%, such as at least 95%, for example at least 97%, such as at least 98%, for example 99% to the sequence of SEQ ID NO: 7.

In another embodiment, the bacteriophage comprising the polynucleotide sequence encoding said at least one quorum sensing molecule inhibitor comprises the sequence set forth in SEQ ID NO: 5 (encoding AqdC) or a functional variant thereof. In one embodiment, the variant sequence encoding AqdC comprises a polynucleotide sequence having a sequence identity of at least 80% to the polynucleotide sequence of SEQ ID NO: 5, such as at least 85%, for example at least 90%, such as at least 95%, for example at least 97%, such as at least 98%, for example 99% to the polynucleotide sequence of SEQ ID NO: 5.

In one embodiment, the anti-CRISPR repressor is encoded by a polynucleotide sequence comprising the sequence set forth in SEQ ID NO:2 or a functional variant of said sequence. In the context of the present invention, the variant of the repressor is a repressor that displays the same activity or essentially the same activity as said anti-CRISPR associated repressor. In one embodiment, the variant sequence encoding the anti-CRISPR associated repressor comprises a sequence having a sequence identity of at least 80% to the polynucleotide sequence of SEQ ID NO: 2, such as at least 85%, for example at least 90%, such as at least 95%, for example at least 97%, such as at least 98%, for example 99% to the polynucleotide sequence of SEQ ID NO: 2.

In another embodiment, the bacteriophage encodes and expresses two inhibitors of quorum sensing molecule, such as two enzymes targeting at least one quorum sensing molecule. In a further embodiment, the bacteriophage encodes and expresses QsdA and AqdC, such as the N-acylhomoserine lactonase (QsdA) from *Rhodococcus erythropolis* and the alkyl quinolone degradation C (AqdC) from *Mycobacteroides abscessus.*

Quorum sensing is the regulation of gene expression in response to fluctuations in cell-population density. Quorum sensing bacteria produce and release signal molecules, quorum sensing molecule (also called autoinducers), that increase in concentration as a function of cell density. Bacteria may for example use the quorum sensing to sense that the population density has reached a threshold level. At this stage, the bacteria communicate through the quorum sensing molecules, which enable them to express genes for different phenotypes, especially those responsible for their virulent behaviour. Thus upon reaching a threshold concentration, quorum sensing molecules trigger cascades of signal transduction which are well described in many bacterial species and regulate processes such as biofilm formation, virulence, competence and sporulation

Some bacterial species also seem to use quorum sensing to enhance each other's virulence, such as *Burkholderia cepacia* and *Pseudomonas aeruginosa,* which sometimes co-infect the lungs of cystic fibrosis patients.

The bacteriophage of the present invention, which express at least one QS inhibitor, while maintaining the capacity of lysing the host cells is a promising alternative to attenuating pathogenicity. While the target bacteria may escape infection by developing resistance, such phage-resistant bacteria are still targets for inhibition by the QS inhibitor, which suppress virulence.

The inventors have demonstrated that the bacteriophage of the present invention effectively limits virulence factor production in co-cultures of phage-susceptible and phage-resistant populations (*see* Examples and **Fig. 7****).**

In the context of the present invention, a quorum sensing molecule is a bacterially synthesized molecule that it either passively or actively released into the extracellular space and involved in quorum sensing. Classes of quorum sensing molecules includes but are limited to acylated homoserine lactones, and oligopeptides.

In one embodiment, the quorum sensing molecule is selected from the group consisting of N-acyl homoserine lactones including harveyi autoinducer 1 (HAI-1), Pseudomonas Quinolone Signal 2-heptyl-3-hydroxy-4-quinolone (PQS), 4-hydroxy-2-heptylquinoline (HHQ), 4-hydroxy-2-heptylquinoline-N-oxide (HQNO), autoinducer-2 ((3aS,6S,6aR)-2,2,6,6a-Tetrahydroxy-3a-methyltetrahydro-2H-furo[2,3-d][1,3,2]dioxaborol-2-uide), Auto inducing peptides (AIP), diffusible signal factor (DSF, cis-11-methyldodecenoicacid), methyl 3-hydroxymyristate (3-OH MAME) or methyl 3-hydroxypalmitate (3-OH PAME), cholerae autoinducer 1 (S)-3-hydroxytridecan-4-one (CAI-1), , and 3,5-dimethyl-pyrazin-2-ol (DPO).

In the context of the present invention, the term host cell refers to a bacterium that is susceptible to infection by the bacteriophage of the present invention. In one embodiment, the bacterium is a pathogenic bacterium. In another embodiment, the bacteriophage is capable of infecting at least one bacteria selected from the group consisting of a *Pseudomonas* sp, such as *Pseudomonas aeruginosa, Enterococcus faecium, Staphylococcus aureus, Klebsiella pneumoniae, Acinetobacter baumannii, Enterobacter* spp., *Escherichia coli, Ralstonia solanacearum, Pseudomonas syringae, Xanthomonas campestris, Xylella fastidiosa, Dickeya dadantii, Agrobacterium tumefasciens,* and *Pectobacterium atrosepticum.*

In another embodiment, the bacterium is a probiotic bacterium. The probiotic bacterium may be used as "carrier" of the QS inhibitor. The probiotic bacterium carrying the bacteriophage of the present invention may thus be used to combat bacterial infection by sole inhibiting quorum sensing.

### Compositions comprising the bacteriophage of the present invention

In another aspect, the present invention provides a composition comprising the bacteriophage of the present invention. The composition is typically prepared not to include ingredients that affect or significantly affect the viability of the bacteriophage. In fact, the composition may include one or more ingredients that stabilizes the bacteriophage and increases the shelf life of the bacteriophage in the composition. The composition may be in dry form, semi dry or liquid form.

The composition may comprise a combination of bacteriophage of the present invention. The combination of bacteriophage may be targeting the same or different bacteria. In another embodiment, combination of bacteriophage encodes different inhibitors of quorum sensing molecules. Accordingly, a composition comprising a combination of different bacteriophage may be useful for targeting a combination of pathogenic bacterial species and/or intervening with quorum sensing by inhibiting or eliminating more than one quorum sensing molecule of the system.

Ingredients that stabilize the bacteriophage may be a sugar, such as a sugar selected from the group consisting of N-acetyl-D-glucosamine, 2-deoxy-D-glucose, maltose, L-rhamnose, cellobiose, and D-xylose.

The titer of the bacteriophage in the composition is typically at least 10⁵ pfu/ml, such as at least 10⁷ pfu/ml, for example at least 10⁹ pfu/ml, such as in the range of 10¹⁰ - 10¹² pfu/ml.

In a further aspect, the composition is a pharmaceutical composition comprising the bacteriophage composition and at least one pharmaceutical acceptable excipient. The pharmaceutical compositions of the present invention can be prepared by admixing a quantity of the bacteriophages with a pharmaceutically acceptable carrier.

The pharmaceutical composition may for example be formulated for oral administration, topical administration (such as transdermal administration or administration to the eyes), pulmonary administration, intranasal administration, rectal administration or intravenous administration.

For example, the composition of the present invention may be for administration in the form of an injectable composition. A typical composition for such purpose comprises a pharmaceutically acceptable carrier. For instance, the composition may contain about 10 mg of human serum albumin per millilitre of phosphate buffer containing NaCl. When the composition comprises a stabilizing sugar, the sugar concentration should be adapted to reach a non-toxic concentration as known to one skilled in the art.

Pharmaceutically acceptable carriers/excipients include aqueous solutions, non-toxic excipients, including salts, preservatives, buffers and the like, as described in Remington's Pharmaceutical Sciences, 15th Ed. Easton: Mack Publishing Co. pp 1405-1412 and 1461-1487 (1975) And The National Formulary XIV., 14th Ed. Washington: American Pharmaceutical Association (1975). Examples of non-aqueous solvents include propylene glycol, polyethylene glycol, vegetable oil and injectable organic esters such as ethyloleate. Aqueous carriers can include water, alcoholic/aqueous solutions, saline solutions, parenteral vehicles such as sodium chloride, Ringer's dextrose, and the like. Intravenous vehicles include fluid and nutrient replenishers. Preservatives include antimicrobials, anti-oxidants, chelating agents and inert gases. The pH and exact concentration of the various components of the bacteriophage pharmaceutical compositions of the invention can be adjusted according to routine known in the art.

Alternatively, the pharmaceutical compositions of the present invention can be in the form of liposomes, lipophilic microcapsules, dendrimers or the like for oral administration to treat systemic bacterial infections. Those skilled in the art are capable of preparing the compositions of the present invention in the form of a lipophilic microcapsule, a dendrimer or a liposome using conventional techniques known in the art.

### Use of anti-QS bacteriophage

One aspect of the present invention provides that bacteriophage of the present invention for use as a medicament.

In particular, the bacteriophage of the present invention or a composition comprising said bacteriophage is provided for use in treating bacterial-infectious diseases. Thus, in one embodiment, the bacteriophage or a composition comprising said bacteriophage for use in the treatment of a bacterial infection.

The compositions of the present invention can be used to treat mammals, such as humans, having bacterial infections. Suitable bacteriophage-containing compositions can be prepared that will be effective in killing, obliterating or reducing the quantity of any of the bacterial microorganisms using the guidelines presented above.

The present invention further provides a method for treating bacterial infection in a subject, comprising administering to the subject a pharmaceutical composition consisting of a therapeutically effective amount of the bacteriophage of the present or a pharmaceutically composition comprising said bacteriophage and at least one pharmaceutical excipient.

In the context of the present invention, the expression "treating bacterial infections," denotes either (i) killing or obliterating sufficient bacterial microorganisms to render the microorganisms ineffective in infecting the host, or (ii) reducing a sufficient quantity of bacterial microorganisms so as the render the microorganisms more susceptible to treatment using conventional antibiotics.

In one embodiment, the bacteriophage is for use in the treatment of a bacterial infection selected from the group consisting of a *Pseudomonas* sp infection, such as a *Pseudomonas aeruginosa* infection, *Enterococcus faecium, Staphylococcus aureus, Klebsiella pneumoniae, Acinetobacter baumannii, Enterobacter spp, Escherichia coli, Neisseria gonorrhoeae, Neisseria meningitidis, Streptococcus, Clostridium species, Bacillus cereus, Mycobacterium bovis, Brucella species, Yersinia enterocolitica, Listeria monocytogenes, Campylobacter,* and *Salmonella.*

*Clostridium species, Bacillus cereus, Mycobacterium bovis, Brucella species, Yersinia enterocolitica, Listeria monocytogenes, Campylobacter,* or *Salmonella* infections are typically found in bovine species, porcine species and poultry. Milk and meat product obtained from the infected animals may thus be the source of infection of human beings, who have consumed or have been in contacted with infected matter. Accordingly, the bacteriophage of the present invention may be used for treating infections in the animals and/or humans infected with the pathogen.

In one embodiment, the subject to be treated for the bacterial infection is a livestock, such as a cow, a sheep, a pig or a chicken. The subject may also be a pet animal, such as a dog or a cat.

In another embodiment, the subject to be treat for a bacterial infection is a human subject.

Bacterial infections comprising *Pseudomonas* sp infection, such as a *Pseudomonas aeruginosa* infection, *Enterococcus faecium, Staphylococcus aureus, Klebsiella pneumoniae, Acinetobacter baumannii, Enterobacter spp, Escherichia coli, Neisseria gonorrhoeae, Neisseria meningitidis,* or *Streptococcus* are typically transmitted from humans to humans either directly or indirectly (for example through waste material comprising the pathogen).

ESKAPE pathogens refers to the scientific names of six highly virulent and antibiotic resistant bacterial pathogens including: *Enterococcus faecium, Staphylococcus aureus, Klebsiella pneumoniae, Acinetobacter baumannii, Pseudomonas aeruginosa,* and *Enterobacter* spp.

The bacteriophage of the present invention is a new intervention in the treatment of pathogenic bacteria that are resistant to conventional treatment with antibiotics.

In one embodiment, the pathogen is a pathogenic bacterium resistant to antibiotics. In a particular embodiment, pathogen is an ESKAPE pathogens selected from the group consisting of *Enterococcus faecium, Staphylococcus aureus, Klebsiella pneumoniae, Acinetobacter baumannii, Pseudomonas aeruginosa* and *Enterobacter* spp.

In one embodiment, a combination of bacteriophages of the present invention is used for the treatment of for example an infection comprising more than one species of bacteria. A combination of bacteriophages may also be used to intervene with quorum sensing by inhibiting or eliminating more than one quorum sensing molecule of the system.

The bacterial infection may manifest in the form a variety of bacterial infectious diseases.

In one embodiment, the bacterial infection is selected from the group consisting of wounds such as burn wounds, diabetic foot ulcer, a urinary tract infection, gastrointestinal infection, infection of the eyes, pulmonary infections and blood infection.

In another embodiment, the bacterial infection is selected from the group consisting infected medical devices/implants. Non-limiting examples of medical devices/implants includes pacemakers, mechanical cardiac valves, prosthesis such as hip prosthesis and knee prosthesis.

The subject may be a subject that is particularly vulnerable to bacterial infection and in particular infection with an ESKAPE pathogen. Non-limiting examples of these subjects include immunocompromised subjects, preterm infants, diabetic subjects. The subject may also be suffering from an underlying condition such as cystic fibrosis.

The bacteriophage for use according to the present invention may be formulated to the particular infection to be treated. In one embodiment, the bacteriophage is formulated for oral administration, topical administration (such as administration to the eyes), pulmonary administration, rectal administration or intravenous administration.

In a further aspect, the bacteriophage of the present invention is provided for use against a plant pathogenic bacteria.

The present invention further provides a method for treating bacterial infection of a plant, comprising administering to the plant a composition consisting of an effective amount of the bacteriophage of the present invention.

In one embodiment, the plant pathogenic bacteria is selected from the group consisting of *Ralstonia solanacearum, Pseudomonas syringae, Xanthomonas campestris, Xanthomonas axonopodis, Xanthomonas oryzae, Xylella fastidiosa, Dickeya dadantii, Dickeya solani, Agrobacterium* tumefasciens, *Erwinia amylovora, Pectobacterium carotovorum,* and *Pectobacterium atrosepticum.*

The bacteriophage may be formulated as a composition that is suitable for being for example dispersed over a field comprising infected crops. For example, a field of olive trees infected with *Xylella fastidiosa.*

The terms "comprising", "comprise" and "comprises" herein are intended by the inventors to be optionally substitutable with the terms "consisting of", "consist of" and "consists of", respectively, in every instance.

The present invention is further characterized in the following non-limiting embodiments.

Embodiment 1. A bacteriophage encoding and expressing at least one inhibitor of a quorum sensing molecule, wherein the expression of said at least one inhibitor of a quorum sensing molecule is operably linked to a promoter, wherein said promoter is regulated by a repressor.

Embodiment 2. The bacteriophage according to embodiment 1, wherein said promoter regulates transcription of said at least one inhibitor of a quorum sensing molecule and said repressor.

Embodiment 3. The bacteriophage according to any one of embodiments 1 or 2, wherein said at least one inhibitor of a quorum sensing molecule and said repressor are encoded by a bi-cistronic or poly-cistronic transcript.

Embodiment 4. The bacteriophage according to any of one the preceding embodiments, wherein said bacteriophage further encodes and expresses an anti-CRISPR (acr) protein.

Embodiment 5. The bacteriophage according to embodiment 4, wherein said at least one inhibitor of a quorum sensing molecule, said repressor and said an anti-CRISPR (acr) protein are encoded by a poly-cistronic transcript.

Embodiment 6. The bacteriophage according to any of one the preceding embodiments, wherein said promoter is an anti-CRISPR promoter and said repressor is an anti-CRISPR associated repressor.

Embodiment 7. The bacteriophage according to any of one the preceding embodiments comprising an anti-CRISPR operon comprising an anti-CRISPR promoter and encoding said at least one inhibitor of a quorum sensing molecule and a sequence encoding an anti-CRISPR associated repressor.

Embodiment 8. The bacteriophage according to embodiment 7, wherein the nucleotide sequence encoding said at least one inhibitor of a quorum sensing molecule is inserted in the operon replacing or in part replacing the sequence encoding an endogenous anti-crispr (acr) protein.

Embodiment 9. The bacteriophage according to any of one the preceding embodiments, wherein said promoter comprises the sequence set forth in SEQ ID NO: 1.

Embodiment 10. The bacteriophage according to any of one the preceding embodiments, wherein said anti-CRISPR repressor is encoded by a polynucleotide sequence comprising the sequence set forth in SEQ ID NO:2.

Embodiment 11. The bacteriophage according to any of one the preceding embodiments, wherein said anti-CRISPR repressor comprises the amino acid sequence set forth in SEQ ID NO: 3 (anti-CRISPR-associated_repressor_from_phage_DMS3) or amino acid sequence having at least 85% identity to the amino acid sequence set forth in SEQ ID NO: 3.

Embodiment 12. The bacteriophage according to any of one the preceding embodiments comprising an endogenous anti-CRISPR operon, wherein said operon is genetically modified to encode and express said at least one inhibitor of a quorum sensing molecule.

Embodiment 13. The bacteriophage according to any of one the preceding embodiments comprising an exogenous anti-CRISPR operon, wherein said operon is genetically modified to encode and express said at least one inhibitor of a quorum sensing molecule.

Embodiment 14. The bacteriophage according to any of one the preceding embodiments, wherein said bacteriophage is lytic.

Embodiment 15. The bacteriophage according to any of one the preceding embodiments, wherein said bacteriophage is temperate.

Embodiment 16. The bacteriophage according to any of one the preceding embodiments, wherein said at least one quorum sensing molecule inhibitor is selected from the group consisting of quorum sensing molecule inhibitor, quorum sensing molecule degrading enzymes, and a repressor of said quorum sensing molecule.

Embodiment 17. The bacteriophage according to any of one the preceding embodiments, wherein said at least one quorum sensing molecule inhibitor is an enzyme selected from the group consisting of quorum sensing molecule degrading enzymes such as alkylquinolone degradation C (AqdC), autoinducing peptide (AIP)-degrading proteases, AHL-degrading enzymes such as AHL lactonases and acylases (e.g. autoinducer inactivation A (AiiA), autoinducer inactivation B (AiiB), autoinducer inactivation C (AiiC), autoinducer inactivation D (AiiD), autoinducer inactivation S (AiiS), attachment gene M (AttM), N-acylhomoserine lactone D (AhlD), N-acylhomoserine lactone K (AhlK), N-acylhomoserine lactone M (AhlM), N-acylhomoserine lactone S (AhlS), metallo-β-lactamase superfamily lactonase (AaL), quorum-sensing signal degradation A (QsdA), quorum-sensing signal degradation H (QsdH), autoinducers degrading hydrolase (AidH), phosphotriesterase-like lactonase (MCP), quorum-quenching lactonase from *Geobacillus kaustophilus* (GKL), NAHL-lactonase (QlcA), quorum signal utilization and inactivation protein (QuiP), pyoverdine synthesis Q (PvdQ), AHL acylase A (HacA), Phosphotriesterase-Like Lactonase SacPox, lactonase SsoPox, and AHL acylase B (HacB).

Embodiment 18. The bacteriophage according to any of one the preceding embodiments, wherein said repressor of said quorum sensing molecule is selected from the group consisting of a synthase synthesizing said repressor of said quorum sensing molecule, an antisense RNA or CRISPRi repressing expression said quorum sensing molecule.

Embodiment 19. The bacteriophage according to any of one the preceding embodiments, wherein said quorum sensing molecule is selected from the group consisting of N-acyl homoserine lactones including harveyi autoinducer 1 (HAI-1), Pseudomonas Quinolone Signal 2-heptyl-3-hydroxy-4-quinolone (PQS), 4-hydroxy-2-heptylquinoline (HHQ), 4-hydroxy-2-heptylquinoline-N-oxide (HQNO), autoinducer-2 ((3aS,6S,6aR)-2,2,6,6a-Tetrahydroxy-3a-methyltetrahydro-2H-furo[2,3-d][1,3,2]dioxaborol-2-uide), Auto inducing peptides (AIP), diffusible signal factor (DSF, cis-11-methyldodecenoicacid), methyl 3-hydroxymyristate (3-OH MAME) or methyl 3-hydroxypalmitate (3-OH PAME), cholerae autoinducer 1 (CAI-1), , and 3,5-dimethyl-pyrazin-2-ol (DPO).

Embodiment 20. The bacteriophage according to any of one the preceding embodiments, wherein the sequence encoding said at least one quorum sensing molecule inhibitor comprises the sequence set forth in SEQ ID NO: 4 (encoding QsdA).

Embodiment 21. The bacteriophage according to any of one the preceding embodiments, wherein the sequence encoding said at least one quorum sensing molecule inhibitor comprises the sequence set forth in SEQ ID NO: 5 (encoding AqdC).

Embodiment 22. The bacteriophage according to any of one the preceding embodiments, wherein said at least one quorum sensing molecule inhibitor comprises the amino acid sequence set forth in SEQ ID NO: 6 (QsdA) or amino acid sequence having at least 85% identity to the amino acid sequence set forth in SEQ ID NO: 6.

Embodiment 23. The bacteriophage according to any of one the preceding embodiments, wherein said at least one quorum sensing molecule inhibitor comprises the amino acid sequence set forth in SEQ ID NO: 7 (AqdC) or amino acid sequence having at least 85% identity to the amino acid sequence set forth in SEQ ID NO: 7.

Embodiment 24. The bacteriophage according to any of one the preceding embodiments, wherein said bacteriophage is capable of infecting at least one bacteria selected from the group consisting of a *Pseudomonas* sp, such as *Pseudomonas aeruginosa, Enterococcus faecium, Staphylococcus aureus, Klebsiella pneumoniae, Acinetobacter baumannii, Enterobacter* spp., *Escherichia coli, Ralstonia solanacearum, Pseudomonas syringae, Xanthomonas campestris, Xylella fastidiosa, Dickeya dadantii, Agrobacterium tumefasciens,* and *Pectobacterium atrosepticum.*

Embodiment 25. The bacteriophage according to any of one the preceding embodiments, wherein said bacteriophage is selected from the listing consisting of Phage DMS3 (accession_no.NC_008717.1), Phage JBD44 (accession_no.NC_030929.1), and phages classified in the orders of *Caudoviricetes, Kalamavirales, Mindivirales, Timlovirales, Tubulavirales, Vinavirales,* and *Durnavirales,* and unclassified or reclassified phages.

Embodiment 26. The bacteriophage according to any of one the preceding embodiments for use as a medicament.

Embodiment 27. The bacteriophage according to any of one the preceding embodiments for use in the treatment of a bacterial infection.

Embodiment 28. The bacteriophage according to any of one the preceding embodiments for use in the treatment of a bacterial infection selected from the group consisting of a *Pseudomonas* sp infection, such as a *Pseudomonas aeruginosa* infection, *Enterococcus faecium, Staphylococcus aureus, Klebsiella pneumoniae, Acinetobacter baumannii, Enterobacter spp, Escherichia coli, Neisseria gonorrhoeae, Neisseria meningitidis, Streptococcus, Clostridium species, Bacillus cereus, Mycobacterium bovis, Brucella species, Yersinia enterocolitica, Listeria monocytogenes, Campylobacter,* and *Salmonella*

Embodiment 29. The bacteriophage for use according to any one of embodiments 27 and 28, wherein said infection is selected from the group consisting of infected medical devices/implants, wounds such as burn wounds, diabetic foot ulcer, a urinary tract infection, gastrointestinal infection, eye infection, pulmonary infections and blood infection.

Embodiment 30. The bacteriophage for use according to any one of embodiments 27 to 29, wherein the subject is an immunocompromised subject, preterm infant, diabetic subject.

Embodiment 31. The bacteriophage for use according to any one of embodiments 27 to 30, wherein the subject is a human subject.

Embodiment 32. The bacteriophage for use according to any one of embodiments 27 to 31, wherein said bacteriophage is formulated for oral administration, topical administration (such as administration to the eyes), pulmonary administration, rectal administration, or intravenous administration.

Embodiment 33. A composition comprising the bacteriophage according to any one of embodiments 1 and 25.

Embodiment 34. A pharmaceutical composition comprising the bacteriophage according to any one of embodiments 1 and 25 and at least one pharmaceutical acceptable excipient.

Embodiment 35. Use of bacteriophage according to any of one the preceding embodiments against a plant pathogenic bacteria.

Embodiment 36. The use according to embodiment 35, wherein said plant pathogenic bacteria is selected from the group consisting of *Ralstonia solanacearum, Pseudomonas syringae, Xanthomonas campestris, Xanthomonas axonopodis, Xanthomonas oryzae, Xylella fastidiosa, Dickeya dadantii, Dickeya solani, Agrobacterium* tumefasciens, *Erwinia amylovora, Pectobacterium carotovorum,* and *Pectobacterium atrosepticum.*

### Examples

### Results

*P. aeruginosa* is often associated with hospital-acquired infections and poses an urgent global health risk. The pathogen communicates using AIs to coordinate behavior such as biofilm formation and virulence in addition to launching anti-phage defense systems. Thus, an effective anti-bacterial strategy is to inhibit AIs, thereby disarming the pathogen without killing it. Another promising antimicrobial approach is the use of phages to infect and kill bacteria.

The inventors engineered the *P. aeruginosa* phage JBD44 (accession no. NC_030929.1) (SEQ ID NO: 11) and phage DMS3 (accession no. NC_008717.1) (SEQ ID NO: 10) to express the N-acylhomoserine lactonase QsdA, which cleaves AHLs **(Example 1B),** and the PQS dioxygenase AqdC that cleaves PQS **(Example 1D)** and reduces *P. aeruginosa* virulence **(Examples 2-3).** To ensure rapid and potent *qsdA* and *aqdC* expression, the inventors cloned them under the anti-CRISPR promoter and controlled by the anti-CRISPR-associated repressor. The DMS3 phage naturally encodes the anti-QS peptide Aqs1 that binds and inhibits LasR. To exclusively assess the anti-QS effect of expressing AI-degrading enzymes from DMS3, the inventors additionally mutated the *aqs1* gene from DMS3, preventing Aqs1 from binding and blocking LasR.

The inventors used a *Galleria mellonella,* wax moth larvae burn wound model of *P. aeruginosa* infection, since it closely mimics the infection dynamics of mammalian infection models. This model offers a robust *in vivo* infection system and it is ideal for the purpose of comparing the effectiveness of different therapies against *P. aeruginosa* infection.

First, the inventors addressed if the anti-QS effect of the engineered phages could rescue *G*. *mellonella* wax moth larvae from *P. aeruginosa* infection in a lysogenic state *i.e*. without phage-mediated killing of *P. aeruginosa* **(Examples 2-3).** The P*acr* promoter used in the engineered phages drives early and rapid expression upon phage infection but is then repressed and controlled by the Aca repressor. To mimic an acute phage infection expression level of anti-QS genes, the inventors used the P*acr* promoter but without the Aca repressor to control expression in a JBD44 lysogen of *P. aeruginosa* UCBPP-PA14. This allows for high anti-QS gene expression in the lysogenic state of the prophage. To quantify the ability of the anti-QS constructs in the engineered phages to inhibit *P. aeruginosa* virulence, the inventors used a recently established *G. mellonella* larvae *in vivo* burn wound infection model. Strikingly, the QsdA and AqdC expressing anti-QS JBD44 prophage effectively disarmed *P. aeruginosa,* resulting in similar survival rates as uninfected controls after 5 days **(****Fig. 2****).** In stark contrast, none of the larvae infected with *P. aeruginosa* carrying the wildtype (WT) JBD44 prophage survived 4 days post infection **(****Fig. 2****).** Thus, expressing AI-degrading enzymes from a prophage was sufficient to render *P. aeruginosa* harmless and protect *G*. *mellonella* larvae.

To further investigate the ability of the anti-QS phages to rescue *G*. *mellonella* from *P. aeruginosa* infection, the inventors infected the larvae with PA14 overnight culture instead of PA14 JBD44 lysogens **(Examples 2-3).** One hour after initiating PA14 infection, the inventors subjected the larvae to various DMS3 phage treatments to test the effect of phage infection with the parental DMS3 aqsl_FSDARE vir phage or phage infection with a 1:1 mixture of virulent DMS3 phages expressing *qsdA* or *aqdC,* respectively. Similarly, to what the inventors had observed with the anti-QS JBD44 lysogens, anti-QS phage treatment with *qsdA-* and aqdC-carrying DMS3 vir phages resulted in significantly higher survival rates as compared to phage treatment with the parental DMS3 aqsl_FSDARE vir phage **(****Fig. 3****).** This further demonstrates the potential of the anti-QS phages to protect *G*. *mellonella* from *P. aeruginosa* infection.

Phage-resistant mutants are naturally selected for in populations during phage exposure. Therefore, the inventors challenged the robustness of the anti-QS phages to inhibit QS activation in mixed populations of phage-susceptible and phage-resistant cells. As phage receptors perform key functions in bacteria, including virulence, the inventors created a phage-resistant mutant without deleting the phage receptor to avoid adverse effects. Instead, the inventors generated a JBD44 prophage lacking the ability to create phage particles, to provide phage-resistance. Specifically, the inventors deleted the JBD44 genes *gpl-39* in a PA14 JBD44 lysogen. This strain, PA14 JBD44 Δ*gp1-39,* was resistant to JBD44 phage infection, unlike the WT PA14 strain **(****Fig. 4****).** Likewise, the inventors created a PA14 DMS3 aqsl_FSDARE Δ*gp26-48* lysogen, resistant to DMS3 vir infection. To confirm that it was resistant, DMS3 vir was applied in a line on a vertical line on a plate and PA14 WT and the PA14 DMS3 aqsl_FSDARE Δ*gp26-48* lysogen were streaked across the line of phage, resulting in killing of PA14 WT only **(****Fig. 5****).** The inventors then tested the ability of the DMS3 anti-QS phage to degrade QS molecules in a mixed population of phage-susceptible and phage-resistant *P. aeruginosa* cells. Specifically, the inventors infected co-cultures of phage-susceptible and phage-resistant cells at a 9-to-1 ratio with a multiplicity of infection (MOI) of 10 and quantified the relative QS molecule levels. 3O-C12-HSL and PQS levels were significantly reduced in response to anti-QS phage infection **(Example 6).** The inventors additionally tested the ability of the JBD44 anti-QS phage to effectively silence and/or kill mixed populations of phage-susceptible and phage-resistant *P. aeruginosa* cells. Specifically, the inventors infected co-cultures of phage-susceptible and phage-resistant cells at a 9-to-1 ratio with an MOI of 5 and incubated for 5.5 h to achieve phage infection of susceptible cells, while allowing phage-resistant cells to grow, reach high cell densities to activate QS, and produce the QS-activated virulence factor pyocyanin **(Example 7).** Remarkably, the anti-QS phage significantly inhibited pyocyanin accumulation by 7.8-fold and reduced the cell density of the mixed populations by 2.5-fold compared to the WT phage **(****Fig. 7****),** implying that the anti-QS phage enabled sufficient QsdA and AqdC enzyme production to degrade 3OC12-HSL and PQS and thereby ultimately limiting pyocyanin accumulation. Thus, the anti-QS phage effectively inhibited the growth of mixed populations of phage-susceptible and phage-resistant cells in addition to disarming the emerging phage-resistant population, underlining that phages encoding quorum sensing molecule degrading enzymes may be highly potent for phage therapy.

Phage infection of *P. aeruginosa* can trigger a specific phage defense mediated by a stress response in which the infected cells overproduce PQS. Healthy nearby *P. aeruginosa* cells which are motile through a process known as swarming sense the PQS signal released by the infected cells, and these motile swarm cells redirect to avoid colliding with the infected cells. The inventors tested the effect of the anti-QS phages to inhibit PQS production during phage-infection of bacterial colonies and thereby avoid repulsion of uninfected cells in a swarming assay. Whereas infection by the phage that does not inhibit the PQS-caused repulsion of the uninfected swarm cells 4 out of 4 times, the anti-QS phage-infected colonies were unable to repulse healthy swarm cells, which collided with the uninfected swarms in every scenario **(Example 8).** Thus, the anti-QS phages effectively inhibit a physical phage defense mechanism, known as the collective stress response, by limiting PQS accumulation.

### Discussion

The inventors present engineered anti-QS phages as precision antimicrobials that combine the unconventional treatment options of quorum quenching and phage therapy to treat *P. aeruginosa* infections in *G. mellonella* wax moth larvae **(****Fig. 2-3****).** This synergistic combination allows specific pathogen killing alongside production and release of the anti-QS enzymes QsdA and AqdC at the site of infection. This approach may enhance the antimicrobial effect of phages by silencing the bacteria evolving immunity to the phages, as AI degrading enzymes released from infected phage-sensitive bacteria degrade the extracellular AIs emitted by any phage-immune bacteria **(****Fig. 6****),** hindering activation of virulence of the remaining cells **(****Fig. 7****).** Additionally, the anti-QS phages inhibit phage defenses, allowing for more efficient phage spread and infection **(Table 2).** In a patient setting, this may pave the way for natural clearance of residual phage-resistant mutants by the human immune system and/or healthy microbiota.

The inventors discovered that the anti-QS enzymes QsdA and AqdC expressed from a prophage as well as from virulent phages disarm *P. aeruginosa* when infecting *G. mellonella* larvae **(****Fig. 2-3****)** and that the anti-QS phages effectively limit virulence factor production in co-cultures of phage-susceptible and phage-resistant populations **(****Fig. 7****).** While previous studies have shown that expressing quorum quenching enzymes from plasmids limits *P*. *aeruginosa* virulence effectively, applying purified quorum quenching enzymes to treat *P. aeruginosa.* This limited success could additionally be due to the anti-QS enzymes being degraded or extensively diluted in the animal model before reaching the site of infection. The inventors' approach circumvents these limitations by enabling *in situ* amplification of the "drug" at the site of infection, using a phage specific for the pathogen, in addition to providing phage-mediated clearance of the targeted pathogen.

As QS controls virulence properties in a broad range of pathogens infecting humans, animals, and plants, the concept of combining quorum-modulating proteins and phage therapy may be generalized to target other bacterial pathogens beyond the human pathogen *P. aeruginosa* described here. The principle of arming phages with the ability to manipulate their host bacteria for reduced bacterial virulence and enhanced bacterial killing may pave the way for implementing effective phage therapy to mitigate the potential global disaster of a post antibiotic area.

### Methods

### Example 1

**Bacterial growth conditions.** *P. aeruginosa* and *E. coli* strains were grown at 37 °C with 300 rpm shaking in Luria-Bertani (LB) broth or on LB agar plates containing 1.5% agar (wt/vol). When appropriate, overnight cultures were diluted in fresh LB medium to a lower OD₆₀₀. Bacterial strains, plasmids, and phages used in this study are listed in **Table 1.**

**DNA manipulations.** JBD44 and DMS3 phages were armed with genes encoding the N-acylhomoserine lactonase QsdA from *Rhodococcus erythropolis* (accession no. AP008957.1) and the PQS dioxygenase AqdC from *Mycobacteroides abscessus* (accession no. CP050978.1) using homologous recombination. For phages encoding both enzymes, *qsdA* and *aqdC* were placed in tandem and separated by 3 bp (GTT). For the DMS3 phage, the constructs were inserted downstream of the anti-CRISPR promoter, while deleting the type I-E anti-CRISPR gene (DMS3-30) but leaving the anti-CRISPR-associated repressor gene (DMS3-31) intact in lysogens carrying DMS3 prophages. For the JBD44 phage, the anti-CRISPR expression system was used to control transcription of the anti-QS constructs. These were inserted between JBD44 *gp46* and *gp47* and the Lambda T1 transcriptional terminator was included immediately downstream of the inserts. Details are outlined below.

Plasmids were extracted using NucleoSpin^{®} Plasmid EasyPure kit (Machery-Nagel, Ref. 740727.250). Plasmids for cloning were digested with FastDigest^{™} restriction enzymes (Thermo Scientific^{™}) and dephosphorylated using FastAP Thermosensitive Alkaline Phosphatase (Thermo Scientific^{™}, Ref. EF0654) according to manufacturer's instructions. Linearized plasmids were gel-purified using NucleoSpin^{®} Gel and PCR Clean-up kit (Machery-Nagel, Ref. 740609.250). For cloning purposes, PCRs were carried out using the Q5^{®} High Fidelity DNA Polymerase (New England BioLabs, Ref. M0491L). For other purposes, PCRs were performed using the DreamTaq DNA Polymerase (Thermo Scientific^{™}, Ref. EP0702). PCR products were purified using NucleoSpin^{®} Gel and PCR Clean-up kit (Machery-Nagel, Ref. 740609.250). Desired inserts were assembled with digested plasmids using T4 DNA Ligase (Thermo Scientific^{™}, Ref. EL0016) or Gibson assembly using NEBuilder^{®} HiFi DNA Assembly (New England BioLabs, Ref. M5520AA) according to manufacturer's instructions. Plasmids were recovered in One Shot^{™} TOP10 Chemically Competent Cells (Invitrogen, Ref. C404003) according to manufacturer's instructions or in *E. coli* SM10 λ*pir* using TSS chemical competence. Plasmids were confirmed by PCR and sequencing (Eurofins and/or Plasmidsaurus). Sanger sequencing of pEXG2-based plasmids was done using primers NMHK250+251. Sanger sequencing of crRNAs and repair template in pAB01 was done using primers NHMK197+198 and 225+226, respectively.

The pEXG2-based plasmids were conjugated into *P. aeruginosa* strains by mating. Exconjugants were selected on LB containing 30 µg ml⁻¹ gentamicin and 100 µg ml⁻¹ irgasan and cultured at 37 °C overnight. Colonies were grown in LB at 37 °C for 2 h, plated on LB containing 15% sucrose and no NaCl and cultured at 37 °C overnight. Mutants were confirmed by PCR and sequencing (Eurofins).

The pAB01-based plasmids were electroporated into *P. aeruginosa* PA14 prewashed in 300 mM sucrose, and recovered in LB at 37 °C for 1 h and then plated on LB containing 50 µg ml⁻¹ gentamicin.

Plasmid pEXG2-DMS3-*aqs1* *FSDARE* was constructed by PCR amplifying chosen regions from DMS3 phage using primer pairs 119+120 and 121+122 designed with 50 bp overlaps. The fragments were fused using PCR and inserted into BamHI- and HindIII-digested and dephosphorylated pEXG2 using Gibson assembly. The resulting mutations cause a change in Aqs1 amino acid sequence position 39-44 from YRDALD to FSDARE in phage DMS3. The DMS3 prophages were confirmed by PCR and sequencing using primers 19+20 (Eurofins).

Plasmid pEXG2-DMS3-Δ*acrI-E::qsdA-aqdC* was constructed by PCR amplifying chosen regions from DMS3 phage using primer pairs 21+83 and 171+26. The synthesized genes *qsdA* (TAG Copenhagen) and *aqdC* (Eurofins) were PCR amplified using primer pairs 85+82 and 81+172, respectively. The fragments were designed with 50 bp overlaps, fused using PCR and finally inserted into BamHI- and HindIII-digested and dephosphorylated pEXG2 using Gibson assembly. DMS3 prophages were confirmed by PCR and sequencing using primers 27+28+129+172 (Eurofins).

Plasmid pEXG2-DMS3-Δ*acrI-E:*:*aqdC* was constructed by PCR amplifying chosen regions from pEXG2-DMS3-Δ*acrI-E::qsdA-aqdC* using primer pairs 21+22 and 23+26 designed with 50 bp overlap. The fragments were fused using PCR and inserted into BamHI- and HindIII-digested and dephosphorylated pEXG2 using Gibson assembly. DMS3 prophages were confirmed by PCR and sequencing using primers 27+28 (Eurofins).

Plasmid pEXG2-DMS3-Δ*acrI-E*::*aqdA* was constructed by PCR amplifying chosen regions from pEXG2-DMS3-Δ*acrI-E::qsdA-aqdC* using primer pairs 21+183 and 184+26 designed with 50 bp overlap. The fragments were fused using PCR and inserted into BamHI- and XhoI-digested and dephosphorylated pEXG2 using Gibson assembly. DMS3 prophages were confirmed by PCR and sequencing using primers 27+28 (Eurofins).

Plasmid pEXG2-DMS3-Δ*gp2p6-48* was constructed by PCR amplifying chosen regions from DMS3 phage using primer pairs 150+151 and 152+153 designed with 50 bp overlaps. The fragments were fused using PCR and inserted into BamHI- and HindIII-digested and dephosphorylated pEXG2 using Gibson assembly. DMS3 prophages were confirmed by PCR and sequencing using primers 154+155 (Eurofins).

Plasmid pAB01 was constructed by using primers 215+216 and plasmid pHERD30T as template in site-directed mutagenesis PCR. Briefly, primers with overlapping regions containing the crRNA insert were used to generate a synthetic plasmid from the template pHERD30T plasmid. The Dam-methylated template plasmid was selectively degraded using DpnI digestion leaving the synthetic plasmid intact. The protocol is based on the paper by Liu and Naismith. The Q5 PCR reactions containing 5% DMSO, the GC enhancer and primers at 0.4 µM each were run on a gradient with annealing temperatures of 55-70 °C and 6 min extension time. Subsequently, the reactions were pooled and DpnI-digested at 37 °C for 4 h. Then, the synthetic plasmid was transformed into *E. coli* as described earlier.

Plasmid pAB01-crRNA-DMS3-*cI* was constructed by phosphorylating primers 217+218 using T4 Polynucleotide Kinase (Thermo Scientific^{™}, Ref. EK0031) according to manufacturer's instructions. Then, the primers were brought to 50 mM NaCl final concentration using a 4 M NaCl stock and annealed at 98 C for 5 min, then gradually cooling down 2 degrees at a time with 30 sec at each temperature. Then, 30 nM of annealed primers were inserted into 40 ng BbsI-digested and dephosphorylated pAB01 using T4 ligase at room temperature overnight.

Plasmid pAB01-DMS3-Δ*cI* was constructed by PCR amplifying chosen regions from phage DMS3 using primer pairs 223+227 and 228+229 designed with 50 bp overlap. The fragments were fused using PCR and inserted into NheI-digested and dephosphorylated pAB01-crRNA-DMS3-*cI* using Gibson assembly. This template can delete bp position 154-380 of DMS3 *cI* repressor (DMS3-1).

Virulent DMS3 phages were constructed by infecting 1:1,000 back-diluted overnight cultures of *P. aeruginosa* PA14 pAB01-DMS3-ΔcI with 200 µl of sterile-filtered supernatants from overnight cultures of various DMS3 lysogens and cultured in 2 ml LB containing 50 µg ml⁻¹ gentamicin and 0.1% arabinose at 37 °C for 5 h. To isolate single plaques, plate lysates were created using 500 µl supernatant and 200 µl uninfected *P. aeruginosa* PA14 pAB01-DMS3-Δ*cI* overnight culture in soft agar containing 50 µg ml⁻¹ gentamicin and 0.1% arabinose and incubated at 37 °C overnight. Virulent DMS3 phages were confirmed by sequencing with primers #6+35 and re-streaked on lawns of PA14 pAB01-DMS3-Δ*cI*.

Cloning of JBD44 prophages and phages was carried out following similar principles and protocols as described above for DMS3 phages.

### Phage infection assays

*P. aeruginosa* PA14 cultures were challenged with DMS3 *aqs1*_FSDARE vir or anti-QS variants. The phages were mixed in a 1:1 ratio for the dual phage treatment. At OD600~0.14, phage-susceptible PA14 was infected with a 10-fold excess of phages (multiplicity of infection = 10). (A) OD₆₀₀ was measured to monitor bacterial growth post phage infection and supernatants were sampled and 0.2-µm sterile-filtered for QS molecule measurements.

### Luminescence reporter assays - QS molecule measurements

To evaluate the relative abundances of the QS molecules 3O-C12-HSL, C4-HSL, and PQS in sterile-filtered culture supernatants, *E. coli* strains carrying an arabinose inducible pBAD expression vector and a pCS26 lux reporter were used. In these strains, arabinose induces production of either the 3O-C12-HSL-responsive LasR, C4-HSL-responsive RhlR, or PQS-responsive PqsR QS receptors driving the expression from the *lasb, rhlA,* or the *pqsA* promoters, respectively, upstream of the *luxCDABE* operon in pCS26. Thus, these strains sense the QS molecules and produce luminescence as a result. Briefly, overnight cultures of the *E. coli* strains carrying the respective plasmids were 100-fold diluted in LB containing 100 µg ml⁻¹ kanamycin, 100 µg ml⁻¹ ampicillin and 0.1% arabinose and 10 µl *P. aeruginosa* sterile-filtered culture supernatants in a total volume of 200 µl per well. For PQS measurements, the *P. aeruginosa* supernatants were 100-fold diluted, whereas for measurements of 3O-C12-HSL and C4-HSL, the supernatants were undiluted. The measurements were carried out in black-well, clear-bottom 96-well plates with intermittent shaking at 30 °C and OD₆₀₀ and luminescence was measured using a Synergy H1 plate reader (BioTek Instruments Inc.) with software Gen5 v.3.05.11. The values are reported as RLU (lum/OD₆₀₀) (relative light units).

### Phage proliferation

Briefly, the engineered phages were purified by re-streaking single plaques on PA14 lawns three times, resuspended in SM buffer, and proliferated using plate lysates with PA14 lawns. In more detail, the single plaques were resuspended in 500 µl SM buffer and after three rounds of re-streaking, 300 µl of the resuspended plaque solution was mixed with 50 µl PA14 overnight culture and 5 ml molten soft agar, subsequently poured onto LB agar plates and incubated at 37 °C overnight. Next, 5 mL SM buffer was added to the infected lawns and incubated for 3-5 h at room temperature. The phages were then collected, 0.2-µm sterile-filtered and enumerated using standard plaque assay on lawns of PA14 or PA14 pAB01-DMS3-ΔcI containing 50 and 200 ul overnight culture, respectively. For lawns of PA14 pAB01-DMS3-Δ*cI*, 50 µg ml⁻¹ gentamicin and 0.1% arabinose were embedded in the soft agar.

### Examples 2-3 - Larvae burn wound infection assay

*Galleria mellonella* wax moth larvae (raised antibiotics free) were ordered from a local pet shop. Once delivered, larvae were immediately stored at 12 °C for no longer than 10 days. Before running an experiment, the larvae were sorted according to size and placed in petri dishes with 10 larvae per dish. The larval bodies were sterilized using 70% ethanol and placed back at 12 °C briefly until further use. A burn wound was created using the head of a stainless-steel nail with a diameter of 2 mm. The nail was heated until red using a Bunsen burner and let to cool down for 4 sec before gently applying the nail head to the back of the larvae for a total of 4 sec. To infect the fresh wounds, 10 µl of OD = 1 standardized inoculum of bacterial overnight culture or undiluted overnight culture were slowly pipetted onto the wound. In addition to untreated larvae and larvae with an uninfected wound, a solvent control was included in which fresh burn wounds were inoculated with PBS. If desired, burn wounds were treated with a total of 10⁹ PFU (plaque forming units) of phage one hour after bacterial infection. The larvae were placed in the 37 °C incubator for up to 5 days and monitored for survival at regular intervals.

### Example 4 - Phage susceptibility test in a liquid infection assay

*P. aeruginosa* PA14 and PA14 lysogen JBD44 Δ*gpl-39* were treated with either SM buffer solvent control or with a MOI 10 of WT JBD44 phage. OD₆₀₀ was measured at 37 °C in a plate reader over a 24 h period.

### Example 5 - Cross streak assay phage susceptibility test

A phage lysate of DMS3 vir was applied in a vertical line across the surface of an agar plate. One dry, a single colony of PA14 and the PA14 DMS3 *aqs1*_FSDARE Δ*gp26-48* lysogen were streaked perpendicularly across the line of phage lysate, exposing these cells to the phage. The plate was incubated overnight at 37 °C.

### Example 6 - Phage infection of phage-susceptible and phage-resistant co-cultures and QS molecule measurement

Phage-susceptible (PA14 WT) and phage-resistant cells (PA14 DMS3 *aqs1*_FSDARE Δ*gp26-48* lysogen) were co-cultured at a ratio of 9-1 and treated with DMS3 aqs1_FSDARE vir phage, as well as phages expressing either of the anti-QS enzymes, or a 1:1 mixture of *aqdC-* and *qsdA*-expressing phages. At OD600~0.14 PA14 co-cultures were infected with a 10-fold excess of phages (multiplicity of infection = 10). (A) OD₆₀₀ was measured to monitor bacterial growth post phage infection. The relative abundance of 3O-C12-HSL, C4-HSL, and PQS were quantified by *E. coli* bioreporters, as described in **Example 1,** producing relative light units as a response to the QS molecules. The values are reported as RLU (lum/OD₆₀₀) (relative light units).

### Example 7 - Phage infection of phage-susceptible and phage-resistant co-cultures and virulence factor measurement

PA14 and PA14 JBD44 Δ*gp1-39* overnight cultures were mixed in a 9-to-1 ratio based on OD₆₀₀, back-diluted to OD₆₀₀=0.01 in LB and grown at 37 °C shaking with 300 rpm. At OD₆₀₀=0.15, the mixed co-cultures were infected with either JBD44 WT phage or phage JBD44 Δ*gp48-53 gp46-47:*:P*acr-qsdA-aqdC-aca* at a MOI of 5. After 5.5 h post infection, pyocyanin levels in supernatants as well as cell densities were measured at OD₆₉₅ and OD₆₀₀, respectively, using a Synergy H1 plate reader (BioTek Instruments Inc.) with software Gen5 v.3.05.11.

### Example 8 - Swarm assay

Swarming petri dishes were prepared with 21 ml of M9 minimum medium supplemented with 1 mM MgSO4, 0.2% glucose, 0.5% Casamino Acids, and 0.5% agar. Petri dishes were dried for 1 h on the bench and for an additional 30 min in a laminar flow hood with the lids off. 5 µl of overnight culture was spotted in the center of the dish, or mixed with 1 µl phage lysate and spotted at satellite positions 2.8 cm from the center spot. Plates were incubated overnight at 37°C in a box containing 10 mL MQ water to maintain humidity.

**Table 1 Strains, phages, and plasmids used in this study**

| **Strain, phage, or plasmid** | **Description** | **Source** |
|---|---|---|
| UCBPP-PA14 | Wildtype *P. aeruginosa* | George O'Toole, Geisel School of Medicine at Dartmouth University, Hanover, NH |
| NMHK342 | PA14 JBD44 lysogen | This study |
| JJM101 | PA14 JBD44 Δ*gp48-53 gp46-47*::P*acr-qsdA-aqdC-T1* | This study |
| JJM178/242 | PA14 JBD44 Δ*gp48-53 gp46-47::*P*acr-qsdA-aqdC-aca-T1* | This study |
| JJM133 | PA14 JBD44 Δ*gp1-39* | This study |
| SMC3884 | PA14 DMS3 lysogen | |
| JJM113 | PA14 DMS3 *aqs1_FSDARE* | This study |
| JJM227 | PA14 DMS3 *aqs1_FSDARE ΔacrIE::qsdA* | This study |
| JJM228 | PA14 DMS3 *aqs1_FSDARE* Δ*acrIE::aqdC* | This study |
| JJM146 | PA14 DMS3 Δ*gp26-48* | This study |
| JJM148 | PA14 DMS3 *aqs1_FSDARE* Δ*gp26-48* | This study |
| *E. coli* SM10 *λpir* | *thi thr leu tonA lacY supE recA::RP4-2-Tc::Mu* | Laboratory stock |
| Plasmid pEXG2 | Allelic exchange vector with pBR origin, gentamicin resistance, *sacB* | |
| pJJM109 | Plasmid pEXG2-JBD44-Δ*gp48-53* | This study |
| pJJM93 | Plasmid pEXG2-JBD44*-gp46-47::Pacr-qsdA-aqdC-T1* | This study |
| pJJM174 | Plasmid pEXG2-JBD44*-gp46-47::Pacr-qsdA-aqdC-aca-T1* | This study |
| pJJM132 | Plasmid pEXG2-JBD44 Δ*gp1-39* | This study |
| pJJM112 | Plasmid pEXG2-DMS3-*aqs1_FSDARE* | This study |
| pJJM239 | Plasmid pEXG2-DMS3-Δ*acrIE::qsdA* | This study |
| pJJM185 | Plasmid pEXG2-DMS3*-*Δ*acrIE::aqdC* | This study |
| pJJM142 | Plasmid pEXG2-DMS3-Δ*gp26-48* | This study |
| pJJM245 | Plasmid pAB01. pHERD30T-based arabinose-inducible expression vector containing a type I-F crRNA insertion site instead of multiple cloning site. Gentamicin resistance. | |
| pJJM247 | Plasmid pAB01-crRNA-DMS3-cI. pAB01 containing crRNA targeting cI repressor in DMS3 phage. | This study |
| pJJM262 | Plasmid pAB01-DMS3-ΔcI | This study |
| Phage JBD44 | Generous gift from Joseph Bondy-Denomy, University of California, San Francisco | |
| Phage JJM178 | Phage JBD44 Δ*gp48-53 gp46-47::Pacr-qsdA-aqdC-aca-T1* | This study |
| Phage DMS3 | Mu-like phage | |
| Phage JJM113 vir | Phage DMS3 *aqs1_FSDARE* vir | This study |
| Phage JJM227 vir | Phage DMS3 *aqs1_FSDARE* Δ*acrIE::qsdA* vir | This study |
| Phage JJM228 vir | Phage DMS3 *aqs1_FSDARE* Δ*acrIE::aqdC* vir | This study |

**Table 2 Anti-QS phages block phage-infection mediated stress response**

| **Satellite colony** | **# Swarm collisions with phage-infected colony** |
|---|---|
| DMS3 *aqs1*_FSDARE vir | 0/4 |
| DMS3 *aqs1*_FSDARE vir *qsdA* | 3/3 |
| DMS3 *aqs1*_FSDARE vir *aqdC* | 4/4 |
| DMS3 *aqs1*_FSDARE vir *qsdA +* DMS3 *aqs1*_FSDARE vir *aqdC* in a 1:1 ratio | 4/4 |

## Claims

1. A bacteriophage encoding and expressing at least one inhibitor of a quorum sensing molecule, wherein the expression of said at least one inhibitor of a quorum sensing molecule is operably linked to a promoter, wherein said promoter is regulated by a repressor.

2. The bacteriophage according to claim 1, wherein said promoter regulates transcription of said at least one inhibitor of a quorum sensing molecule and said repressor.

3. The bacteriophage according to any of one the preceding claims, wherein said promoter is an anti-CRISPR promoter and said repressor is an anti-CRISPR associated repressor.

4. The bacteriophage according to any of one the preceding claims comprising an anti-CRISPR operon comprising an anti-CRISPR promoter and encoding said at least one inhibitor of a quorum sensing molecule and a sequence encoding an anti-CRISPR associated repressor, optionally wherein said operon further encodes an anti-CRISPR (acr) protein.

5. The bacteriophage according to claim 4, wherein the nucleotide sequence encoding said at least one inhibitor of a quorum sensing molecule is inserted in the operon replacing or in part replacing the sequence encoding an endogenous anti-crispr (acr) protein.

6. The bacteriophage according to any of one the preceding claims, wherein said promoter comprises the sequence set forth in SEQ ID NO: 1.

7. The bacteriophage according to any of one the preceding claims, wherein said anti-CRISPR repressor comprises the amino acid sequence set forth in SEQ ID NO: 3 or amino acid sequence having at least 85% identity to the amino acid sequence set forth in SEQ ID NO: 3.

8. The bacteriophage according to any of one the preceding claims, wherein said at least one quorum sensing molecule inhibitor is selected from the group consisting of quorum sensing molecule inhibitor, quorum sensing molecule degrading enzymes, and a repressor of said quorum sensing molecule.

9. The bacteriophage according to any of one the preceding claims, wherein said at least one quorum sensing molecule inhibitor is an enzyme selected from the group consisting of quorum sensing molecule degrading enzymes such as alkylquinolone degradation C (AqdC), autoinducing peptide (AIP)-degrading proteases, AHL-degrading enzymes such as AHL lactonases and acylases (e.g. autoinducer inactivation A (AiiA), autoinducer inactivation B (AiiB), autoinducer inactivation C (AiiC), autoinducer inactivation D (AiiD), autoinducer inactivation S (AiiS), attachment gene M (AttM), N-acylhomoserine lactone D (AhlD), N-acylhomoserine lactone K (AhlK), N-acylhomoserine lactone M (AhlM), N-acylhomoserine lactone S (AhlS), metallo-β-lactamase superfamily lactonase (AaL), quorum-sensing signal degradation A (QsdA), quorum-sensing signal degradation H (QsdH), autoinducers degrading hydrolase (AidH), phosphotriesterase-like lactonase (MCP), quorum-quenching lactonase from *Geobacillus kaustophilus* (GKL), NAHL-lactonase (QlcA), quorum signal utilization and inactivation protein (QuiP), pyoverdine synthesis Q (PvdQ), AHL acylase A (HacA), Phosphotriesterase-Like Lactonase SacPox, lactonase SsoPox, and AHL acylase B (HacB).

10. The bacteriophage according to any of one the preceding claims, wherein said quorum sensing molecule is selected from the group consisting of N-acyl homoserine lactones including harveyi autoinducer 1 (HAI-1), Pseudomonas Quinolone Signal 2-heptyl-3-hydroxy-4-quinolone (PQS), 4-hydroxy-2-heptylquinoline (HHQ), 4-hydroxy-2-heptylquinoline-N-oxide (HQNO), autoinducer-2 ((3aS,6S,6aR)-2,2,6,6a-Tetrahydroxy-3a-methyltetrahydro-2H-furo[2,3-d][1,3,2]dioxaborol-2-uide), Auto inducing peptides (AIP), diffusible signal factor (DSF, cis-11-methyldodecenoicacid), methyl 3-hydroxymyristate (3-OH MAME) or methyl 3-hydroxypalmitate (3-OH PAME), cholerae autoinducer 1 (CAI-1), and 3,5-dimethyl-pyrazin-2-ol (DPO).

11. The bacteriophage according to any of one the preceding claims, wherein said at least one quorum sensing molecule inhibitor comprises the amino acid sequence set forth in SEQ ID NO: 6 (QsdA) or amino acid sequence having at least 85% identity to the amino acid sequence set forth in SEQ ID NO: 6 and/or said at least one quorum sensing molecule inhibitor comprises the amino acid sequence set forth in SEQ ID NO: 7 (AqdC) or amino acid sequence having at least 85% identity to the amino acid sequence set forth in SEQ ID NO: 7.

12. The bacteriophage according to any of one the preceding claims for use as a medicament.

13. The bacteriophage according to any of one the preceding claims for use in the treatment of a bacterial infection, such as an infection is selected from the group consisting of infected medical devices/implants, wounds such as burn wounds, diabetic foot ulcer, a urinary tract infection, gastrointestinal infection, eye infection, pulmonary infections and blood infection, optionally wherein the bacterial infection is selected from the group consisting of a *Pseudomonas* sp infection, such as a *Pseudomonas aeruginosa* infection, *Enterococcus faecium, Staphylococcus aureus, Klebsiella pneumoniae, Acinetobacter baumannii, Enterobacter spp, Escherichia coli, Neisseria gonorrhoeae, Neisseria meningitidis, Streptococcus, Clostridium species, Bacillus cereus, Mycobacterium bovis, Brucella species, Yersinia enterocolitica, Listeria monocytogenes, Campylobacter,* and *Salmonella.*

14. A composition comprising the bacteriophage according to any one of claims 1 and 11, such as a pharmaceutical composition comprising the bacteriophage according to any one of claims 1 and 11 and at least one pharmaceutical acceptable excipient.

15. Use of bacteriophage according to any of one the preceding claims against a plant pathogenic bacteria, such as a plant pathogenic bacteria selected from the group consisting of *Ralstonia solanacearum, Pseudomonas syringae, Xanthomonas campestris, Xanthomonas axonopodis, Xanthomonas oryzae, Xylella fastidiosa, Dickeya dadantii, Dickeya solani, Agrobacterium* tumefasciens, *Erwinia amylovora, Pectobacterium carotovorum,* and *Pectobacterium atrosepticum.*
